# EUROPEAN PATENT SPECIFICATION

(11) **EP 1 450 845 B1**
(45) Date of publication and mention of the grant of the patent: **14.10.2009**
(21) Application number: 02794118.6
(22) Date of filing: 04.12.2002
(51) Int. Cl.: C12Q 1/68, G01N 33/68, A61K 38/16, C07K 14/47, C07K 16/00

(54) **15603, A HUMAN ION CHANNEL FAMILY MEMBER**
15603, EIN MITGLIED DER HUMANEN IONENKANAL-FAMILIE
15603, MEMBRE DE LA FAMILLE DES CANAUX IONIQUES HUMAINS

(30) Priority: 04.12.2001 US 336936 P
(43) Date of publication of application: 01.09.2004
(73) Proprietor: MILLENNIUM PHARMACEUTICALS, INC., Cambridge, MA 02139 (US)
(72) Inventor: GALVIN, Katherine, M., Jamaica Plain, MA 02130 (US)
(74) Representative: Taylor, Kate Laura
(86) International application number: PCT/US2002/038499
(87) International publication number: WO 2003/048188

(56) References cited:
- WO-A-01/30830
- WO-A-98/08979
- WO-A-03/008444
- WO-A1-98/08979
- US-A- 5 932 417
- DATABASE EMBL [Online] 17 December 2003 (2003-12-17), "Sequence 15522 from Patent EP1074617." XP002327086 retrieved from EBI accession no. EM_PRO:AX880617 Database accession no. AX880617
- DATABASE EMBL [Online] 17 December 2003 (2003-12-17), "Sequence 4227 from Patent EP1074617." XP002327087 retrieved from EBI accession no. EM_PAT:AX869322 Database accession no. AX869322
- DATABASE EMBL [Online] 17 December 2003 (2003-12-17), "Sequence 12800 from Patent EP1074617." XP002327088 retrieved from EBI accession no. EM_PAT:AX877895 Database accession no. AX877895
- DATABASE EMBL [Online] 17 December 2003 (2003-12-17), "Sequence 4525 from Patent EP1074617." XP002327089 retrieved from EBI accession no. EM_PAT:AX869620 Database accession no. AX869620
- PHILIPP S. ET AL.: 'TRP4 (CCE1) protein is part of native calcium release-activated Ca2+-like channels in adrenal cells' THE JOURNAL OF BIOLOGICAL CHEMISTRY vol. 275, no. 31, 04 August 2000, pages 23965 - 23972, XP002965570
- HOFMANN T. ET AL.: 'Direct activation of human TRPC6 and TRPC3 channels by diacylglycerol' NATURE vol. 397, 21 January 1999, pages 259 - 263, XP002188162
- DATABASE PROQUEST [Online] D'ESPOSITO M. ET AL.: 'Identification and assignment of a human transient receptor potential channel 6 gene TRPC6 to chromosome 11q21-22', XP002965807 & CYTOGENETICS AND CELL GENETICS vol. 83, no. 1-2, 1998, pages 46 - 47
- BATES D.O. ET AL: 'Regulation of microvascular permeability by vascular endothelial growth factors' JOURNAL OF ANATOMY vol. 200, June 2002, pages 581 - 597, XP009011997

## Description

Many intercellular and intracellular events depend on the regulation of the concentration of certain ions such as calcium, sodium, potassium, and chloride within the cell. Ion channels have been found which regulate the flow of anions or cations through a membrane based on voltage, pH, phosphorylation, and ligand binding. Typically an ion channel consists of multiple transmembrane domains which form a channel through which the ions pass from one side of the membrane to the other. These ion channels may play important roles in how a cell responds to hormones or neurotransmitters with increased activity of enzymes such as phospholipase C and a subsequent rise in the concentration of intracellular free calcium (Ca⁺²). The increase in intracellular calcium concentration may occur as a result of the release of calcium from intracellular stores as well as an influx of calcium through the plasma membrane.

Drosophila transient receptor potential (TRP) proteins and some mammalian homologues (TRPC proteins) are thought to mediate capacitative Ca⁺² entry (Hofmann et al., Nature 397:259-263 (1999)). Seven mammalian homologous genes (TRPC 1 to TRPC 7) have been cloned and characterized. TRPC6, together with TRPC 3 have been identified as the first members of a new functional family of second-messenger-operated cation channels, which are activated by diacylglycerol independently of protein kinase C (Hofmann *et al., supra*)*.* It has recently been demonstrated that TRPC6 is likely to be the essential component of the α1-adrenoceptor-activated nonselective cation channel (α1-AR-NSCC), which may serve as a store-depletion-independent Ca²⁺ entry pathway during increased sympathetic activity. The α1-androgen receptor (α1-AR) is expressed widely in the vascular system. α1-AR stimulation leads to activation of G protein-coupled phospholipase Cβ, which catalyzes formation from phosphoinositide of 1, 4, 5-triphosphate (IP3) and diacylglycerol, which leads to the release of stored Ca²⁺ and sustained Ca²⁺ entry. The present invention relates to a human TRP6, 15603.

The present invention is based, in part, on the discovery of a novel ion channel family member, referred to herein as "15603". The nucleotide sequence of a encoding 15603 is shown in SEQ ID NO:1, and the amino acid sequence of a 15603 polypeptide is shown in SEQ ID NO:2. In addition, the nucleotide sequence of the coding region is depicted in SEQ ID NO:3.

The invention provides methods of screening for compounds that modulate the expression or activity of the 15603 polypeptides or nucleic acids.

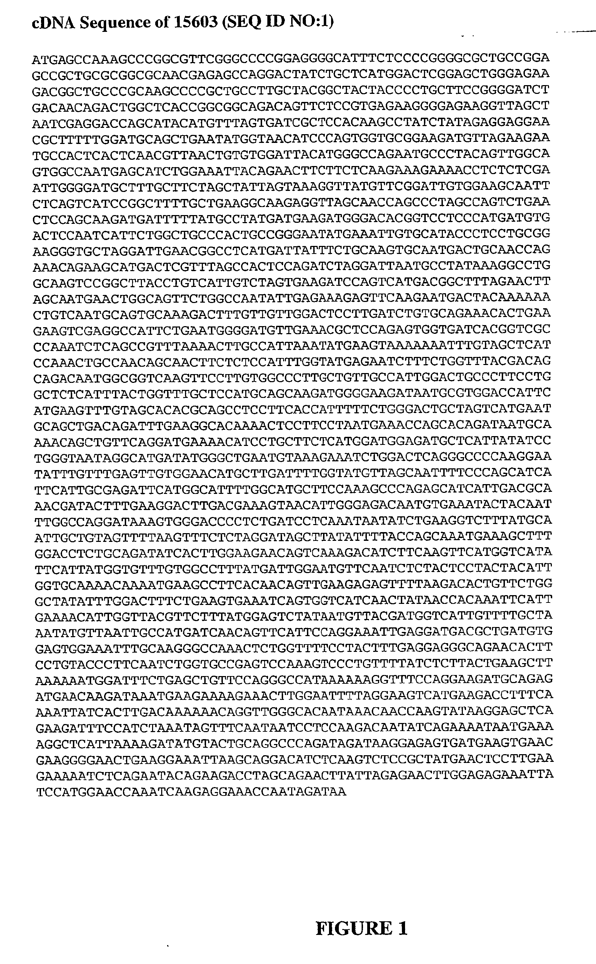

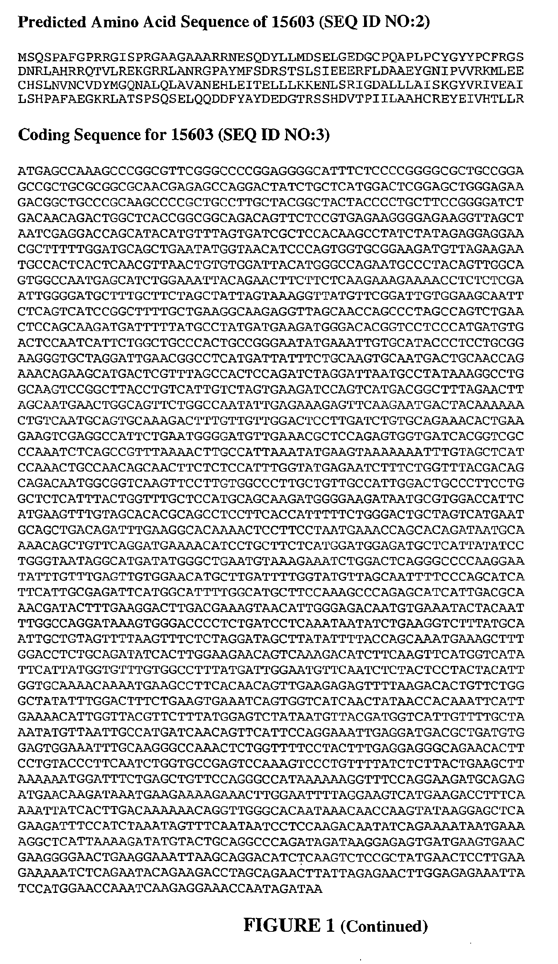

In one aspect the invention provides a method of identifying a compound capable of treating an angiogenic disorder comprising: i) contacting a candidate compound with the nucleic acid molecule of SEQ ID NO:3 as shown in figure 1; and ii) assaying the ability of the compound to inhibit the expression of the nucleic acid molecule, thereby identifying a compound capable of treating an angiogenic disorder.

In an alternative aspect the invention provides a method of identifying a compound capable of treating an angiogenic disorder comprising: i) contacting a candidate compound with a polypeptide encoded by the nucleic acid molecule of SEQ ID NO:3 as shown in figure 1 or a cell expressing the polypeptide encoded by the nucleic acid molecule of SEQ ID NO:3 as shown in figure 1; and ii) assaying the ability of the compound to inhibit the ion channel activity of the polypeptide, thereby identifying a compound capable of treating an angiogenic disorder. In one embodiment said assay is a cell based assay and said candidate compound is contacted with a cell expressing said polypeptide. In alternative embodiments said assay is a cell free assay. In one embodiment said polypeptide is provided as an ion channel in a plasma membrane. In one embodiment said assaying the ability of the compound to inhibit the activity of the polypeptide comprises measuring the flow of ions across said ion channel. In a further embodiment, the flow of ions across said ion channel is measured by patch clamp recording.

In one embodiment the aforementioned methods of identifying a compound capable of treating an angiogenic disorder comprise confirming the ability of a test compound to inhibit the expression of the nucleic acid molecule of SEQ ID NO:3 as shown in figure 1 or to inhibit the polypeptide encoded by the nucleic acid molecule of SEQ ID NO:3 as shown in figure 1 in an animal model.

Other features and advantages of the invention will be apparent from the following detailed description, and from the claims.

### Brief Description of the Drawings

*Figure* 1 depicts a cDNA sequence (SEQ ID NO:1) and predicted amino acid sequence (SEQ ID NO:2) of human 15603. The methionine-initiated open reading frame of human 15603 (without the 5' and 3' untranslated regions of SEQ ID NO:1) is shown also as the coding sequence, SEQ ID NO:3.

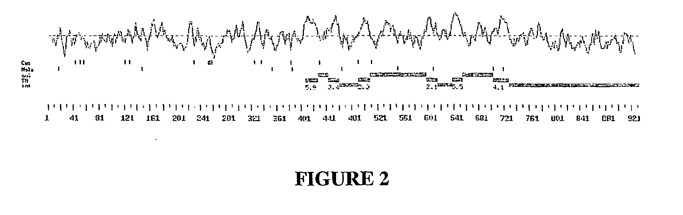

*Figure* 2 depicts a hydropathy plot of human 15603. Relatively hydrophobic residues are shown above the dashed horizontal line, and relatively hydrophilic residues are below the dashed horizontal line. The cysteine residues (cys) are indicated by short vertical lines just below the hydropathy trace The numbers corresponding to the amino acid sequence of human 15603 are indicated- Polypeptides of the invention include fragments which include: all or part of a hydrophobic sequence, *e.g*., a sequence above the dashed line, *e.g*., the sequence from about amino acid 162 to 172, from about amino acid 215 to 225, from about amino acid 325 to 335, from about amino acid 401 to 431, from about amino acid 441 to 461, from about amino acid 486 to 506, from about amino acid 521 to 551, from about amino acid 591 to 616, from about amino acid 631 to 656, from about amino acid 665 to 675, from about amino acid 701 to 731, and from about amino acid 770 to 780 of SEQ ID NO:2; all or part of a hydrophilic sequence, *e.g*., a sequence below the dashed line, *e.g*., the sequence from about amino acid 20 to 32, from about amino acid 55 to 85, from about amino acid 181 to 221, from about amino acid 241 to 281, from, about amino acid 306 to 321, from about amino acid 331 to 340, from about amino acid 461 to 486, from about amino acid 780 to 805, from about amino acid 810 to 841, from about amino acid 841 to 851, from about amino acid 865 to 890, from about amino acid 895 to 905, and from about amino acid 915 to 931 of SEQ ID NO:2; a sequence which includes a Cys, or a glycosylation site.

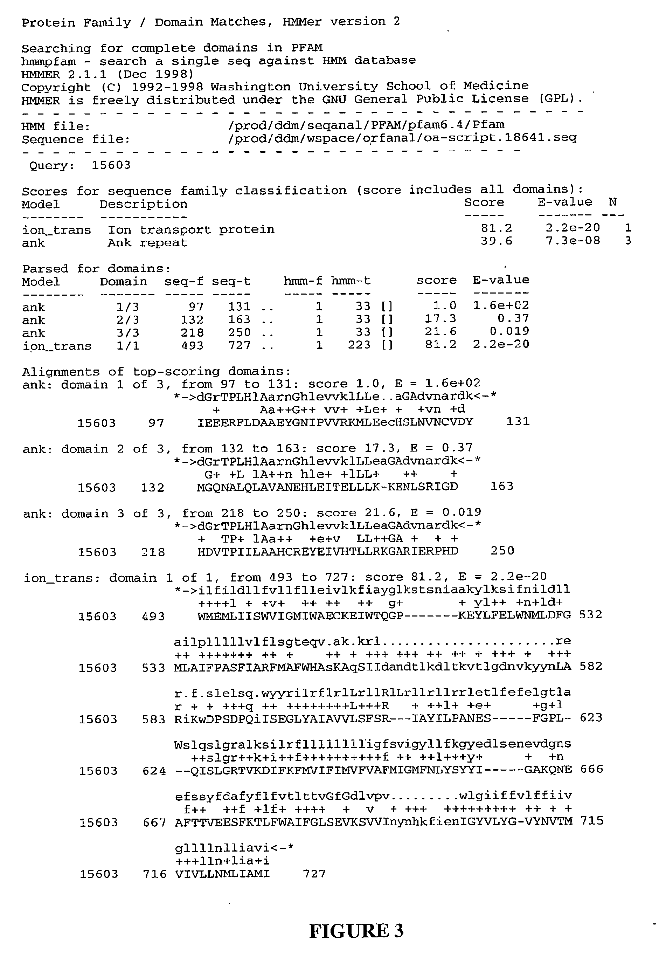

*Figure* 3 depicts an alignment of the ion transport protein and ank repeat domains of human 15603 with a consensus amino acid sequence derived from a hidden Markov model (HMM) from PFAM. The upper sequence is the consensus amino acid sequence (SEQ ID NO:4,5,6) for an ank repeat domain, while the lower amino acid sequences corresponds to amino acids 97 to 131, 132 to 163, and 218 to 250 of SEQ ID NO:2. The consensus amino acid sequence (SEQ ID NO:7) for an ion transport protein domain is also shown aligned with amino acids 493 to 727 of SEQ ID NO:2.

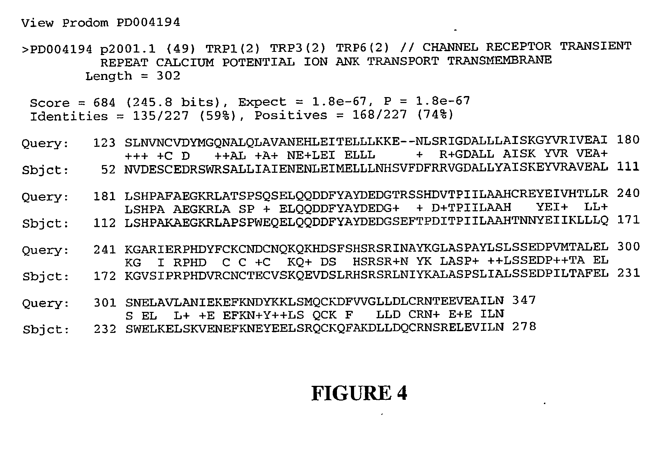

*Figure 4* depicts a BLAST alignment of the human 15603 ion channel domain with a consensus amino acid sequence of a domain derived from the ProDomain database ("Channel Receptor Transient Repeat Calcium Potential Ion Ank Transport Transmembrane;" No. PD004194; ProDomain Release 2001.1; http://www.toulouse.inra.fr/prodom.html). The lower sequence is amino acid residues 52 to 278 of the 278 amino acid PD004194 consensus sequence (SEQ ID NO:8), while the upper amino acid sequence corresponds to the ion channel domain of human 15603, amino acid residues 123 to 347 of SEQ ID NO:2.

### Detailed Description of the Invention

The human 15603 sequence (Figure 1; SEQ ID NO:1), which is approximately 2796 nucleotides long including untranslated regions, contains a predicted methionine-initiated coding sequence of about 2796 nucleotides, including the termination codon (nucleotides indicated as coding of SEQ ID NO:1 in Fig. 1; SEQ ID NO:3). The coding sequence encodes a 931 amino acid protein (SEQ ID NO:2). The human 15603 protein of SEQ ID NO:2 and Figure 2 includes an amino-terminal hydrophobic amino acid sequence, consistent with a signal sequence, of about 26 amino acids (from amino acid 1 to about amino acid 26 of SEQ ID NO:2, PSORT, Nakai and Kanehisa (1992) Genomics 14:897-911), which upon cleavage results in the production of a mature protein form. )). This mature protein form is approximately 905 amino acid residues in length (from about amino acid 27 to amino acid 931 of SEQ ID NO:2).

Human 15603 contains the following regions or other structural features (for general information regarding PFAM identifiers, PS prefix and PF prefix domain identification numbers, refer to Sonnhammer et al. (1997) Protein 28:405-420 and http://www.psc.edu/general/software/packages/pfam/pfam.html):

an ank domain (PFAM Accession Number PF0023) located at about amino acid residues 97 to 131 of SEQ ID NO:2;

an ank domain (PFAM Accession Number PF0023) located at about amino acid residues 132 to 163 of SEQ ID NO:2;

an ank domain (PFAM Accession Number PF0023) located at about amino acid residues 218 to 250 of SEQ ID NO:2;

an ion transport protein domain (PFAM Accession Number PF00520) located at about amino acid residues 493 to 727 of SEQ ID NO:2;

a Receptor Channel Potential Transient Repeat Variant Calcium Entry Ionic domain (ProDom No. PD 140156) located at about amino acid residues 1 to 39 of SEQ ID NO:2;

a Channel Receptor Transient Potential Calcium Repeat Variant Entry Ion domain (ProDom No. PD323618) located at about amino acid residues 40 to 71 of SEQ ID NO:2;

a Channel Receptor Calcium Potential Capacitative Entry Trp-Related Transient Repeat Variant domain (ProDom No. PD186301) located at about amino acid residues 93 to 133 of SEQ ID NO:2;

a Repeat Channel Ionic Receptor Trp Vision Transient Potential Ank domain (ProDom No. PD140149) located at about amino acid residues 95 to 154 of SEQ ID NO:2;

a Repeat Ankyrin Channel Gene ORF Family Receptor Ankyrin-Like Factor (ProDom No. PD007334) located at about amino acid residues 102 to 166 of SEQ ID NO:2;

a Ankyrin Repeat Kinase Domain UNC-44 Ankyrin-Related Alternative Glycoprotein EGF-like domain (ProDom No. PD000041) located at about amino acid residues 105 to 188 of SEQ ID NO:2;

a Channel Receptor Transient Repeat Calcium Potential Ion Ank Transport Transmembrane domain (ProDom No. PD004194) located at about amino acid residues 123 to 347 of SEQ ID NO:2;

a Receptor Channel Potential Transient NOMPC TRP2 Y71A12B.4 Y71A12B.E 2-Beta 2-Alpha domain (ProDom No. PD296552) located at about amino acid residues 299 to 509 of SEQ ID NO:2;

a Transmembrane Fis Receptor MTR1 domain (ProDom No. PD039592) located at about amino acid residues 308 to 916 of SEQ ID NO:2;

a Channel Receptor Calcium Repeat Transient Potential Ion Transmembrane Ionic Transport domain (ProDom No. PD328255) located at about amino acid residues 362 to 509 of SEQ ID NO:2;

a Channel Receptor Calcium Transient Potential Repeat Vanilloid Transmembrane Ion Transport domain (ProDom No. PD003230) located at about amino acid residues 409 to 748 of SEQ ID NO:2;

a Channel Receptor Calcium Potential Repeat Transient Ion Ionic Variant Ank domain (ProDom No. PD238062) located at about amino acid residues 510 to 597 of SEQ ID NO:2;

a Channel Receptor Calcium Repeat Potential Transient Capacitative Entry Ion Transport domain (ProDom No. PD342728) located at about amino acid residues 744 to 895 of SEQ ID NO:2;

a Channel Receptor Transient Potential Repeat Calcium Transport Transmembrane Ank Ionic domain (ProDom No. PD004174) located at about amino acid residues 749 to 900 of SEQ ID NO:2;

a Channel Repeat Calcium Ionic Entry Receptor Transient Ank Transport domain (ProDom No. PD266294) located at about amino acid residues 786 to 854 of SEQ ID NO:2;

a Gelsolin-Related domain (ProDom No. PD202783) located at about amino acid residues 792 to 931 of SEQ ID NO:2;

a Coiled Coil Myosin Repeat Chain Heavy Filament Heptad Pattern Muscle domain (ProDom No. PD000002) located at about amino acid residues 796 to 928 of SEQ ID NO:2;

a Channel Receptor Transient Potential Calcium Repeat Variant Entry Ion (ProDom No. PD 137340) located at about amino acid residues 901 to 931 of SEQ ID NO:2;

a transmembrane domain (predicted by MEMSAT, Jones et al. (1994) Biochemistry 33:3038-3049) at about amino acids 407 to 426 of SEQ ID NO:2;

a transmembrane domain (predicted by MEMSAT, Jones et al. (1994) Biochemistry 33:3038-3049) at about amino acids 443 to 459 of SEQ ID NO:2;

a transmembrane domain (predicted by MEMSAT, Jones et al. (1994) Biochemistry 33:3038-3049) at about amino acids 490 to 507 of SEQ ID NO:2;

a transmembrane domain (predicted by MEMSAT, Jones et al. (1994) Biochemistry 33:3038-3049) at about amino acids 598 to 614 of SEQ ID NO:2;

a transmembrane domain (predicted by MEMSAT, Jones et al. (1994) Biochemistry 33:3038-3049) at about amino acids 637 to 653 of SEQ ID NO:2;

a transmembrane domain (predicted by MEMSAT, Jones et al. (1994) Biochemistry 33:3038-3049) at about amino acids 703 to 727 of SEQ ID NO:2;

a coiled coil structure located at about amino acids 296 to 907 (ALELSNELAV ... LEEKSQNTED) of SEQ ID NO:2;

a leucine zipper pattern (Prosite PS00029) located at about amino acids 766 to 787 (LVPSPKSLFYLLLKLKKWISEL) of SEQ ID NO:2;

nine protein kinase C phosphorylation sites (Prosite PS00005) located at about amino acids 14 to 16 (SPR), 89 to 91 (SDR), 563 to 565 (TLK), 630 to 632 (TVK), 674 to 676 (SFK), 769 to 771 (SPK), 836 to 838 (SIR), 893 to 895 (SLR), and 929 to 931 (TNR) of SEQ ID NO:2;

sixteen casein kinase II phosphorylation sites (Prosite PS00006) located at about amino acids 96 to 99 (SIEE), 197 to 200 (SQSE), 216 to 219 (SSHD), 289 to 292 (SSED), 296 to 299 (TALE), 475 to 478 (TSTD), 492 to 495 (SWME), 556 to 559 (SIID), 563 to 566 (TLKD), 571 to 574 (TLGD), 630 to 633 (TVKD), 669 to 672 (TTVE), 730 to 733 (SFQE), 752 to 755 (SYFE), 815 to 818 (SHED), and 839 to 842 (SSED) of SEQ ID NO:2;

two cAMP/cGMP-dependent protein kinase phosphorylation sites (Prosite PS00004) located at about amino acids 67 to 70 (RRQT) and 319 to 322 (KKLS) of SEQ ID NO:2;

three tyrosine phosphorylation site (Prosite PS00007) located at about amino acids 24 to 31 (RRNESQDY), 101 to 108 (RFLDAAEY), and 698 to 705 (KFIENIGY) of SEQ ID NO:2;

two amidation sites (Prosite PS00009) located at about amino acids 75 to 78 (KGRR) and 188 to 191 (EGKR) of SEQ ID NO:2;

nine N-glycosylation sites (Prosite PS00001) located at about amino acids 26 to 29 (NESQ), 157 to 160 (NLSR), 362 to 365 (NLSR), 394 to 397 (NLSG), 473 to 476 (NETS), 561 to 564 (NDTL), 617 to 620 (NESF), 712 to 715 (NVTM), and 728 to 731 (NSSF) of SEQ ID NO:2; and

five N-myristoylation sites (Prosite PS00008) located at about amino acids 17 to 22 (GAAGAA), 213 to 218 (GTRSSH), 504 to 509 (GMIWAE), 661 to 666 (GAKQNE), and 811 to 816 (GILGSH) of SEQ ID NO:2.

The 15603 protein contains a significant number of structural characteristics in common with members of the ion channel family and the ank repeat family. The term "family" when referring to the protein and nucleic acid molecules of the invention means two or more proteins or nucleic acid molecules having a common structural domain or motif and having sufficient amino acid or nucleotide sequence homology as defined herein. Such family members can be naturally or non-naturally occurring and can be from either the same or different species. For example, a family can contain a first protein of human origin as well as other distinct proteins of human origin, or alternatively, can contain homologs of non-human origin, *e.g*., rat or mouse proteins. Members of a family also can have common functional characteristics.

As used herein, the term "ion channel" includes a protein or polypeptide which is capable of regulating the flow of ions such as calcium cations through a channel. The channel may regulate the flow of a particular cation or anion or may be less discriminating and allow multiple types of cations or anions flow through it. The flow of ions may be regulated by the presence or absence of a bound ligand or may be regulated by the phosphorylation state of the channel or another protein associated with the channel. The ion channel protein may undergo a conformational change based on the binding of a ligand, the phosphorylation of a particular residue, or the binding of another protein or biomolecule.

Members of an ion channel family of proteins are characterized by transmembrane domains, cytoplasmic domains, extracellular domains, and may be homodimers, homotrimers, homomultimers, heterodimeric etc. The pore of ion channels are typically formed by multiple transmembrane proteins encoded by the same or different genes. Typically, a hydrophilic transmembrane channel is formed that allows passage of ions from one side of the plasma membrane to the other. Clusters of charged amino acids at the mouth of the channel may increase the selectivity for a particular type of ion, e.g., clusters of negatively charged amino acid residues at the mouth of the channel may exclude negative ions from cation channels.

All ion channel family proteins form water-filled pores across membranes. Ion channel proteins are located in the plasma membrane of animal and plant cells and are further characterized by small highly selective pores that participate in ion transport. Typically, more than 10⁶ ions can pass through such a channel each second. Many ion channels allow specific ions, such as Na⁺, K⁺, Ca²⁺, or Cl⁻, to diffuse down their electrochemical gradients across the lipid bilayer. The ion channel proteins show ion selectivity, permitting some ions to pass but not others. Another feature of ion channel proteins is that they are not continuously open, in contrast to simple aqueous pores. Instead they have "gates," which open briefly and then close again. This opening and closing is usually in response to a specific purtubation of the membrane, such as a change in voltage across the membrane (voltage-gated channels), mechanical stimulation (mechanically-gated channels), or the binding of a signaling molecule (ligand-gated channels). In the case of ligand-gated channels, the signaling ligand can be either an extracellular mediator, such as a neurotransmitter (transmitter-gated channels), an intracellular mediator, such as an ion (ion-gated channels), a nucleotide (nucleotide-gated channels), or a GTP-binding regulatory protein (G-protein-gated channels).

Ion channels are responsible for the electrical excitability of nerve and muscle cells and mediate most forms of electrical signaling in the nervous system. A single nerve cell typically contains more than five kinds of ion channels. However, these ion channels are not restricted to electrically excitable cells. Ion channels are present in all animal cells and are found in plant cells and microorganisms. For example, ion channel proteins propagate the leaf-closing response of the mimosa plant and allow the single-celled paramecium to reverse direction after collision. 15603, also known as TRPC6, is a non-selective cation channel that is activated by diacylglycerol in a membrane-delimited fashion, independently of protein kinase C

A 15603 polypeptide can include a "ion channel domain" or regions homologous with a "ion channel domain". A 15603 polypeptide can further include a "ank domain" or regions homologous with a "ank domain".

As used herein, the term "ion transport protein domain" includes an amino acid sequence of about 200 to 250 amino acid residues in length and having a bit score for the alignment of the sequence to the ion transport protein domain (HMM) of at least 75. Preferably a ion transport domain mediates regulates the flow of ion through a membrane. Preferably, a ion transport protein domain includes at least about 200 to 300 amino acids, more preferably about 200 to 250 amino acid residues, or about 210 to 240 amino acids and has a bit score for the alignment of the sequence to the ion transport protein domain (HMM) of at least 50, 75, 80, or greater. The ion transport domain can include transmembrane domains. The ion transport protein domain (HMM) has been assigned the PFAM Accession Number PF00520 (http;//genome.wustl.edu/Pfam/.html). An alignment of the ion transport protein domain (amino acids 493 to 727 of SEQ ID NO:2) of human 15603 with the Pfam ion transport protein consensus amino acid sequence (SEQ ID NO:7) derived from a hidden Markov model is depicted in Figure 3.

As used herein, the term "ank repeat domain" includes an amino acid sequence of about 30 to 35 amino acid residues in length and having a bit score for the alignment of the sequence to the ank repeat domain (HMM) of at least 15. Preferably, an ank repeat domain includes at least about 20 to 40 amino acids, more preferably about 25 to 35 amino acid residues, or about 30 to 35 amino acids and has a bit score for the alignment of the sequence to the ank repeat domain (HMM) of at least 10, 15, 20, or greater. The ank repeat domain (HMM) has been assigned the PFAM Accession Number PF0023 (http;//genome.wustl.edu/Pfam/.html). Additionally, the ank repeat domain (HMM) has been assigned the SMART identifier ANK_2a (http://smart.embl-heidelberg.de/). An alignment of the ank repeat domain (amino acids 97 to 131, 132 to 163, and 218 to 250 of SEQ ID NO:2) of human 15603 with the Pfam ank repeat domain consensus amino acid sequence (SEQ ID NO:4,5,6) derived from a hidden Markov model is depicted in Figure 3. An alignment of the ank repeat domain (amino acids 97 to 126, 132 to 160, and 218 to 247 of SEQ ID NO:2) of human 15603 with the SMART ANK_2a domain consensus amino acid sequence (SEQ ID NO:4,5,6) derived from a hidden Markov model is depicted in Figure 3.

Preferably, a 15603 polypeptide or protein has a "ion transport protein domain" or a region which includes at least about 200 to 300 more preferably about 200 to 250 or 210 to 240 amino acid residues and has at least about 60%, 70% 80% 90% 95%, 99%, or 100% homology with a "ion transport protein domain," *e.g*., the ion transport protein domain of human 15603 (*e.g*., residues 493 to 727 of SEQ ID NO:2).

Preferably, a 15603 polypeptide or protein has an "ank repeat domain" or a region which includes at least about 25 to 40 more preferably about 25 to 35 or 30 to 35 amino acid residues and has at least about 60%, 70% 80% 90% 95%, 99%, or 100% homology with an "ank repeat domain," *e.g*., an ank repeat domain of human 15603 (*e.g*., residues 97 to 131, 132 to 163, or 218 to 250 of SEQ ID NO:2).

To identify the presence of a "ion transport protein" domain or an "ank repeat" domain in a 15603 protein sequence, and make the determination that a polypeptide or protein of interest has a particular profile, the amino acid sequence of the protein can be searched against the Pfam database of HMMs (*e.g*., the Pfam database, release 2.1) using the default parameters (http://www.sanger.ac.uk/Software/Pfam/HMM_search). For example, the hmmsf program, which is available as part of the HMMER package of search programs, is a family specific default program for MILPAT0063 and a score of 15 is the default threshold score for determining a hit. Alternatively, the threshold score for determining a hit can be lowered (*e.g*., to 8 bits). A description of the Pfam database can be found in Sonhammer et al. (1997) Proteins 28:405-420 and a detailed description of HMMs can be found, for example, in Gribskov et al. (1990) Meth. Enzymol 183:146-159; Gribskov et al. (1987) Proc. Natl. Acad. Sci. USA 84:4355-4358; Krogh et al. (1994) J. Mol. Biol. 235:1501-1531; and Stultz et al. (1993) Protein Sci. 2:305-314. A search was performed against the HMM database resulting in the identification of a "ion transport protein domain" domain in the amino acid sequence of human 15603 at about residues 493 to 727 of SEQ ID NO:2 (see Figure 1). A search was performed against the HMM database resulting in the identification of three "ank repeat" domains in the amino acid sequence of human, 15603 at about residues 97 to 131, 132 to 163, and 218 to 250 of SEQ ID NO:2 (see Figure 1).

An additional method to identify the presence of an "ank repeat" domain in a 15603 protein sequence, and make the determination that a polypeptide or protein of interest has a particular profile, the amino acid sequence of the protein can be searched against a SMART database (Simple Modular Architecture Research Tool, http://smart.embl-heidelberg.de/) of HMMs as described in Schultz et al. (1998), Proc. Natl. Acad. Sci. USA. 95:5857 and Schultz et al. (2000) Nucl. Acids Res 28:231. The database contains domains identified by profiling with the hidden Markov models of the HMMer² search program (Durbin et al. (1998) Biological sequence analysis: probabilistic models of proteins and nucleic acids. Cambridge University Press.; http://hmmer.wustl.edu/). The database also is extensively annotated and monitored by experts to enhance accuracy. A search was performed against the HMM database resulting in the identification of three "ank 2a" domains in the amino acid sequence of human 15603 at about residues 97 to 126, 132 to 160, and 218 to 247 of SEQ ID NO:2 (see Figure 1).

For further identification of domains in a 15603 protein sequence, and make the determination that a polypeptide or protein of interest has a particular profile, the amino acid sequence of the protein can be searched against a database of domains, *e.g*., the ProDom database (Corpet et al. (1999), Nucl. Acids Res. 27:263-267). The ProDom protein domain database consists of an automatic compilation of homologous domains. Current versions of ProDom are built using recursive PSI-BLAST searches (Altschul et al. (1997) Nucleic Acids Res. 25:3389-3402; Gouzy et al. (1999) Computers and Chemistry 23:333-340) of the SWISS-PROT 38 and TREMBL protein databases. The database automatically generates a consensus sequence for each domain. A BLAST search was performed against the HMM database resulting in the identification of a "channel receptor transient repeat calcium potential ion ank transport transmembrane" domain in the amino acid sequence of human 15603 at about residues 123 to 347 of SEQ ID NO:2 (see Figure 1).

A 15603 molecule can further include a transmembrane domain or an ank repeat domain.

A 15603 polypeptide can include at least one, two, three, four, five, preferably six "transmembrane domains" or regions homologous with "transmembrane domains". As used herein, the term "transmembrane domain" includes an amino acid sequence of about 10 to 40 amino acid residues in length and spans the plasma membrane. Transmembrane domains are rich in hydrophobic residues, *e.g*., at least 50%, 60%, 70%, 80%, 90%, 95% or more of the amino acids of a transmembrane domain are hydrophobic, *e.g*., leucines, isoleucines, tyrosines, or tryptophans. Transmembrane domains typically have alpha-helical structures and are described in, for example, Zagotta et al., (1996) Annual Rev. Neurosci. 19:235-263. The transmembrane domains of human 15603 are located at about residues 407 to 426, 443 to 459,490 to 507, 598 to 614, 637 to 653, and 703 to 727 of SEQ ID NO:2.

In a preferred embodiment, a 15603 polypeptide or protein has at least one, two, three, four, five, preferably six "transmembrane domain," or a region which includes at least about 12. to 35 more preferably about 14 to 30 or 15 to 25 amino acid residues and has at least about 60%, 70% 80% 90% 95%, 99%, or 100% homology with a "transmembrane domain," *e.g*., the transmembrane domains of human 15603 *(e.g.,* residues 407 to 426, 443 to 459, 490 to 507, 598 to 614, 637 to 653, or 703 to 727 of SEQ ID NO:2). The transmembrane domain of human 15603 is visualized in the hydropathy plot (Figure 2) as regions of about 15 to 25 amino acids where the hydropathy trace is mostly above the horizontal line.

To identify the presence of a "transmembrane" domain in a 15603 protein sequence, and make the determination that a polypeptide or protein of interest has a particular profile, the amino acid sequence of the protein can be analyzed by a transmembrane prediction method that predicts the secondary structure and topology of integral, membrane proteins based on the recognition of topological models (MEMSAT, Jones et al., (1994) Biochemistry 33:3038-3049).

A 15603 polypeptide can include at least one, two, three, four, five, six, preferably seven "non-transmembrane regions." As used herein, the term "non-transmembrane region" includes an amino acid sequence not identified as a transmembrane domain. The non-transmembrane regions in 15603 are located at about amino acids 1 to 406, 427 to 442, 460 to 489, 508 to 597, 615 to 636, 654 to 702, and 727 to 931 of SEQ ID NO:2. Non-transmembrane domains can be cytoplasmic or extracellular.

The non-transmembrane regions of 15603 include at least one, two, three, preferably four cytoplasmic regions. When located at the N-terminus, the cytoplasmic region is referred to herein as the "N-terminal cytoplasmic domain." As used herein, an "N-terminal cytoplasmic domain" includes an amino acid sequence having about 1 to 500, preferably about 1 to 450, more preferably about 1 to 425, or even more preferably about 1 to 410 amino acid residues in length, is located inside of a cell or within the cytoplasm of a cell. The C-terminal amino acid residue of an "N-terminal cytoplasmic domain" is adjacent to an N-terminal amino acid residue of a transmembrane domain in a 15603 protein. For example, an N-terminal cytoplasmic domain is located at about amino acid residues 1 to 406 of SEQ ID NO:2.

In a preferred embodiment, a 15603 polypeptide or protein has an N-terminal cytoplasmic domain or a region which includes about 1 to 450, preferably about 1 to 425, and more preferably about 1 to 410 amino acid residues and has at least about 60%, 70% 80% 90% 95%, 99%, or 100% homology with an "N-terminal cytoplasmic domain," *e.g*., the N-terminal cytoplasmic domain of human 15603 (*e.g*., residues 1 to 406 of SEQ ID NO:2).

In another embodiment, a 15603 cytoplasmic region includes at least one, preferably two cytoplasmic loops. As used herein, the term "loop" includes an amino acid sequence which is not included within a phospholipid membrane, having a length of at least about 4, preferably about 5 to 95, more preferably about 6 to 35 amino acid residues, and has an amino acid sequence that connects two transmembrane domains within a protein or polypeptide. Accordingly, the N-terminal amino acid of a loop is adjacent to a C-terminal amino acid of a transmembrane domain in a 15603 molecule, and the C-terminal amino acid of a loop is adjacent to an N-terminal amino acid of a transmembrane domain in a 15603 molecule. As used herein, a "cytoplasmic loop" includes a loop located inside of a cell or within the cytoplasm of a cell. For example, a "cytoplasmic loop" can be found at about amino acid residues 460 to 489 and 615 to 636 of SEQ ID NO:2.

In a preferred embodiment, a 15603 polypeptide or protein has a cytoplasmic loop or a region which includes at least about 4, preferably about 5 to 40, and more preferably about 6 to 35 amino acid residues and has at least about 60%, 70% 80% 90% 95%, 99%, or 100% homology with a cytoplasmic loop," *e.g*., a cytoplasmic loop of human 15603 (*e.g*., residues 460 to 489 and 615 to 636 of SEQ ID NO:2).

In another embodiment, a 15603 non-transmembrane region includes at least one, two, preferably three non-cytoplasmic loops. As used herein, a "non-cytoplasmic loop" includes a loop located outside of a cell or within an intracellular organelle. Non-cytoplasmic loops include extracellular domains (*i.e*., outside of the cell) and intracellular domains (*i.e*., within the cell). When referring to membrane-bound proteins found in intracellular organelles (*e.g*., mitochondria, endoplasmic reticulum, peroxisomes microsomes, vesicles, endosomes, and lysosomes), non-cytoplasmic loops include those domains of the protein that reside in the lumen of the organelle or the matrix or the intermembrane space. For example, a "non-cytoplasmic loop" can be found at about amino acid residues 427 to 442, 508 to 597, and 654 to 702 of SEQ ID NO:2.

In a preferred embodiment, a 15603 polypeptide or protein has at least one non-cytoplasmic loop or a region which includes at least about 4, preferably about 5 to 100, more preferably about 6 to 90 amino acid residues and has at least about 60%, 70% 80% 90% 95%,99%, or 100% homology with a "non-cytoplasmic loop," *e.g*., at least one non-cytoplasmic loop of human 15603 (*e.g*., residues 427 to 442, 508 to 597, and 654 to 702 of SEQ ID NO: 2).

In another embodiment, a cytoplasmic region of a 15603 protein can include the C-terminus and can be a "C-terminal cytoplasmic domain," also referred to herein as a "C-terminal cytoplasmic tail." As used herein, a "C-terminal cytoplasmic domain" includes an amino acid sequence having a length of at least about 200, preferably about 150 to 250, more preferably about 175 to 225 amino acid residues, is located inside of a cell or within the cytoplasm of a cell. The N-terminal amino acid residue of a "C-terminal cytoplasmic domain" is adjacent to a C-terminal amino acid residue of a transmembrane domain in a 15603 protein. For example, a C-terminal cytoplasmic domain is located at about amino acid residues 728 to 931 of SEQ ID NO:2.

In a preferred embodiment, a 15603 polypeptide or protein has a C-terminal cytoplasmic domain or a region which includes at least about 200, preferably about 150 to 250, and more preferably about 175 to 225 amino acid residues and has at least about 60%, 70% 80% 90% 95%, 99%, or 100% homology with a C-terminal cytoplasmic domain," *e.g.,* the C-terminal cytoplasmic domain of human 15603 (*e.g*., residues 728 to 931 of SEQ ID NO:2).

A human 15603 protein can further include a leucine zipper or coiled coil structure. Preferably a leucine zipper domain has at least about 60%, 70%, 80%, 90%, 95%, 99%, or 100% homology with the leucine zipper domain of human 15603 (*e.g*., residues 766 to 787 of SEQ ID NO:2). Preferably a coiled coil domain has at least about 60%, 70%, 80%, 90%, 95%, 99%, or 100% homology with the coiled coil domain of human 15603 (*e.g*., residues 296 to 907 of SEQ ID NO:2). A human 15603 protein can further include N-glycosylation sites, cAMP and cGMP-dependent protein kinase phosphorylation sites, protein kinase C phosphorylation sites, casein kinase II phosphorylation site, tyrosine kinase phosphorylation sites, N-myristoylation sites, and amidation sites.

A 15603 family member can include at least one ion channel protein domain; and at least one, two, preferably three ank repeat domains or transmembrane or non-transmembrane domains. A 15603 family member can include at least one leucine zipper structure or at least one coiled coil structure. Furthermore, a 15603 family member can include at least one, two, three, four, five, six, seven, eight, preferably nine protein kinase C phosphorylation sites (Prosite PS00005); at least one, two, three, four, five, six, seven, eight, nine, ten, eleven, twelve, thirteen, fourteen, fifteen, and preferably sixteen casein kinase II phosphorylation sites (Prosite PS00006); at least one, two, three, four, five, six, seven, eight, preferably nine N-glycosylation sites (Prosite PS00001); at least one, preferably two cAMP/cGMP protein kinase phosphorylation sites (Prosite PS00004); at least one, preferably two amidation sites (Prosite PS00009); and at least one, two, three, four, and preferably five N-myristoylation sites (Prosite PS00008).

As the 15603 polypeptides of the invention can modulate 15603-mediated activities, they can be useful for developing novel diagnostic and therapeutic agents for ion channel-associated or other 15603-associated disorders, as described below.

As used herein, a "ion channel-associated activity" includes an activity which involves the regulation of the flow of ions across a membrane. The flow of ions may be controlled by a second-messenger such as diacylglycerol. Members of the family can play a role in cardiovascular disease such as abnormal angiogenesis.

As used herein, a "15603 activity", "biological activity of 15603" or "functional activity of 15603", refers to an activity exerted by a 15603 protein, polypeptide or nucleic acid molecule on *e.g*., a 15603-responsive cell or on a 15603 substrate, *e.g*., a protein substrate, as determined *in vivo* or *in vitro.* In one embodiment, a 15603 activity is a direct activity, such as an association with a 15603 target molecule. A "target molecule" or "binding partner" is a molecule with which a 15603 protein binds or interacts in nature. In an exemplary embodiment, 15603 is a ion channel, *e.g*., a non-selective cation channel that is activated by diacylglycerol, and thus interacts in nature with a molecule such as a second messenger (*e.g*., diacylglycerol) to regulate the flow of cations through a membrane.

. A 15603 activity can also be an indirect activity, *e.g*., a cellular signaling activity mediated by interaction of the 15603 protein with a 15603 receptor. Based on the above-described sequence structures and similarities to molecules of known function, the 15603 molecules of the present invention can have similar biological activities as ion channel family members. For example, the 15603 proteins of the present invention can have one or more of the following activities: (1) the ability to bind a second messenger; (2) the ability to bind diacylglycerol; (3) the ability to regulate the flow of cations through a membrane; (4) the ability to regulate angiogenesis; and (5) the ability to regulate intracellular calcium levels.

The 15603 molecules of the invention can modulate the activities of cells in tissues where they are expressed. For example, 15603 mRNA is expressed in moderate to high levels in hemangiomas, megakaryocytes, Wilm's tumor, vascular smooth muscle cells, proliferative endothelial cells, uterine, and normal brain cortex. Accordingly, the 15603 molecules of the invention can act as therapeutic or diagnostic agents for cardiovascular, angiogenic, or neoproliferative disorders.

Thus, the 15603 molecules can act as novel diagnostic targets and therapeutic agents for controlling one or more cardiovascular, angiogenic, neoproliferative, /or other ion channel disorders. As used herein, "ion channel disorders" are diseases or disorders whose pathogenesis is caused by, is related to, or is associated with aberrant or deficient ion channel protein function or expression.

The 15603 molecules can be used to treat cardiovascular, immune system, or proliferative disorders in part because family members are found in the hemangiomas, blood vessels, and megakaryocytes.

As used herein, an "endothelial cell disorder" includes a disorder characterized by aberrant, unregulated, or unwanted endothelial cell activity, *e.g*. angiogenesis; or aberrant expression of cell surface adhesion molecules or genes associated with angiogenesis, *e.g*., TIE-2, FLT and FLK. Endothelial cell disorders include tumorigenesis, tumor metastasis, psoriasis, diabetic retinopathy, endometriosis, Grave's disease, ischemic disease (*e.g*., atherosclerosis), and chronic inflammatory diseases (*e.g*., rheumatoid arthritis).

The 15603 protein, fragments thereof, and derivatives and other variants of the sequence in SEQ ID NO:2 thereof are collectively referred to as "15603 polypeptide or proteins". Nucleic acid molecules encoding such polypeptides or proteins are collectively referred to as "15603 nucleic acids."

As used herein, the term "nucleic acid molecule" includes DNA molecules *(e.g.,* a cDNA or genomic DNA) and RNA molecules (*e.g*., an mRNA) and analogs of the DNA or RNA generated, *e.g*., by the use of nucleotide analogs. The nucleic acid molecule, can be single-stranded or double-stranded, but preferably is double-stranded DNA.

The term "isolated or purified nucleic acid molecule" includes nucleic acid molecules which are separated from other nucleic acid molecules which are present in the natural source of the nucleic acid. For example, with regards to genomic DNA, the term "isolated" includes nucleic acid molecules which are separated from the chromosome with which the genomic DNA is naturally associated. Preferably, an "isolated" nucleic acid is free of sequences which naturally flank the nucleic acid (i.e., sequences located at the 5' and/or 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb or 0.1 kb of 5' and/or 3' nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material or culture medium when produced by recombinant techniques, or substantially free of chemical precursors or other chemicals when chemically synthesized.

As used herein, the term "hybridizes under low stringency, medium stringency, high stringency, or very high stringency conditions" describes conditions for hybridization and washing. Guidance for performing hybridization reactions can be found in Current Protocols in Molecular Biology (1989) John Wiley & Sons, N.Y., 6.3.1-6.3.6. Aqueous and nonaqueous methods are described in that reference and either can be used. Specific hybridization conditions referred to herein are as follows: 1) low stringency hybridization conditions in 6X sodium chloride/sodium citrate (SSC) at about 45°C, followed by two washes in 0.2X SSC, 0.1% SDS at least at 50°C (the temperature of the washes can be increased to 55°C for low stringency conditions); 2) medium stringency hybridization conditions in 6X SSC at about 45°C, followed by one or more washes in 0.2X SSC, 0.1% SDS at 60°C; 3) high stringency hybridization conditions in 6X SSC at about 45°C, followed by one or more washes in 0.2X SSC, 0.1% SDS at 65°C; and preferably 4) very high stringency hybridization conditions are 0.5M sodium phosphate, 7% SDS at 65°C, followed by one or more washes at 0.2X SSC, 1% SDS at 65°C. Very high stringency conditions (4) are the preferred conditions and the ones that should be used unless otherwise specified.

As used herein, a "naturally-occurring" nucleic acid molecule refers to an RNA or DNA molecule having a nucleotide sequence that occurs in nature (*e.g*., encodes a natural protein).

As used herein, the terms "gene" and "recombinant gene" refer to nucleic acid molecules which include an open reading frame encoding a 15603 protein, preferably a mammalian 15603 protein, and can further include non-coding regulatory sequences, and introns.

An "isolated" or "purified" polypeptide or protein is substantially free of cellular material or other contaminating proteins from the cell or tissue source from which the protein is derived, or substantially free from chemical precursors or other chemicals when chemically synthesized. In one embodiment, the language "substantially free" means preparation of 15603 protein having less than about 30%, 20%, 10% and more preferably 5% (by dry weight), of non-15603 protein (also referred to herein as a "contaminating protein"), or of chemical precursors or non-15603 chemicals. When the 15603 protein or biologically active portion thereof is recombinantly produced, it is also preferably substantially free of culture medium, i.e., culture medium represents less than about 20%, more preferably less than about 10%, and most preferably less than about 5% of the volume of the protein preparation. The invention includes isolated or purified preparations of at least 0.01, 0.1, 1.0, and 10 milligrams in dry weight.

A "non-essential" amino acid residue is a residue that can be altered from the wild-type sequence of 15603 (*e.g*., the sequence of SEQ ID NO:1 or 3) without abolishing or more preferably, without substantially altering a biological activity, whereas an "essential" amino acid residue results in such a change. For example, amino acid residues that are conserved among the polypeptides of the present invention, *e.g*., those present in the ion channel protein or ank repeat domains, are predicted to be particularly unamenable to alteration.

A "conservative amino acid substitution" is one in which the amino acid residue is replaced with an amino acid residue having a similar side chain. Families of amino acid residues having similar side chains have been defined in the art. These families include amino acids with basic side chains (*e.g*., lysine, arginine, histidine), acidic side chains (*e.g*., aspartic acid, glutamic acid), uncharged polar side chains (*e.g*., glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g*., threonine, valine, isoleucine) and aromatic side chains (*e.g*., tyrosine, phenylalanine, tryptophan, histidine). Thus, a predicted nonessential amino acid residue in a 15603 protein is preferably replaced with another amino acid residue from the same side chain family. Alternatively, in another embodiment, mutations can be introduced randomly along all or part of a 15603 coding sequence, such as by saturation mutagenesis, and the resultant mutants can be screened for 15603 biological activity to identify mutants that retain activity. Following mutagenesis of SEQ ID NO:1 or SEQ ID NO:3, the encoded protein can be expressed recombinantly and the activity of the protein can be determined.

As used herein, a "biologically active portion" of a 15603 protein includes a fragment of a 15603 protein which participates in an interaction between a 15603 molecule and a non-15603 molecule. Biologically active portions of a 15603 protein include peptides comprising amino acid sequences sufficiently homologous to or derived from the amino acid sequence of the 15603 protein, *e.g*., the amino acid sequence shown in SEQ ID NO:2, which include fewer amino acids than the full length 15603 protein, and exhibit at least one activity of a 15603 protein. Typically, biologically active portions comprise a domain or motif with at least one activity of the 15603 protein, *e.g*., ability to regulate cation flow through a membrane. A biologically active portion of a 15603 protein can be a polypeptide which is, for example, 10, 25, 50, 100, 200 or more amino acids in length. Biologically active portions of a 15603 protein can be used as targets for developing agents which modulate a 15603 mediated activity, *e.g*., an ability to regulate cation flow through a membrane.

Calculations of homology or sequence identity (the terms "homology" and "identity" are used interchangeably herein) between sequences are performed as follows:

To determine the percent identity of two amino acid sequences, or of two nucleic acid sequences, the sequences are aligned for optimal comparison purposes (*e.g*., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, 90%, 100% of the length of the reference sequence (*e.g*., when aligning a second sequence to the 15603 amino acid sequence of SEQ ID NO:2 having 931 amino acid residues, at least 30%, preferably at least 40%, more preferably at least 50%, even more preferably at least 60%, and even more preferably at least 70%, 80%, or 90% amino acid residues are aligned). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch (1970) J. Mol. Biol. 48:444-453 algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used if the practitioner is uncertain about what parameters should be applied to determine if a molecule is within a sequence identity or homology limitation of the invention) are a Blossum 62 scoring matrix with a gap penalty of 12, a gap extend penalty of 4, and a frameshift gap penalty of 5.

The percent identity between two amino acid or nucleotide sequences can be determined using the algorithm of Meyers and Miller ((1989) CABIOS, 4:11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul et al. (1990) J. Mol. Biol. 215:403-10. BLAST nucleotide searches can be performed with the NBLAST program, score = 100, wordlength = 12 to obtain nucleotide sequences homologous to 15603 nucleic acid molecules of the invention. BLAST protein searches can be performed with the XBLAST program, score = 50, wordlength = 3 to obtain amino acid sequences homologous to 15603 protein molecules of the invention. To obtain gapped alignments for comparison purposes, Gapped BLAST can be utilized as described in Altschul et al., (1997) Nucleic Acids Res. 25:3389-3402. When utilizing BLAST and Gapped BLAST programs, the default parameters of the respective programs (*e.g*., XBLAST and NBLAST) can be used. See http://www.ncbi.nlm.nih.gov.

Particular 15603 polypeptides have an amino acid sequence substantially identical to the amino acid sequence of SEQ ID NO:2. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. For example, amino acid sequences that contain a common structural domain having at least about 60%, or 65% identity, likely 75% identity, more likely 85%, 90%. 91%, 92%, 93%, 94%, 95%, 96%, 97%, 98% or 99% identity to SEQ ID NO:2 are termed substantially identical.

In the context of nucleotide sequence, the term "substantially identical" is used herein to refer to a first nucleic acid sequence that contains a sufficient or minimum number of nucleotides that are identical to aligned nucleotides in a second nucleic acid sequence such that the first and second nucleotide sequences encode a polypeptide having common functional activity, or encode a common structural polypeptide domain or a common functional polypeptide activity. For example, nucleotide sequences having at least about 60%, or 65% identity, likely 75% identity, more likely 85%, 90%. 91%, 92%, 93%, 94%, 95%, 96%, 97%, 98% or 99% identity to SEQ ID NO:1 or 3 are termed substantially identical.

"Misexpression or aberrant expression", as used herein, refers to a non-wild type pattern of gene expression, at the RNA or protein level. It includes: expression at non-wild type levels, i.e., over or under expression; a pattern of expression that differs from wild type in terms of the time or stage at which the gene is expressed, *e.g*., increased or decreased expression (as compared with wild type) at a predetermined developmental period or stage; a pattern of expression that differs from wild type in terms of decreased expression (as compared with wild type) in a predetermined cell type or tissue type; a pattern of expression that differs from wild type in terms of the splicing size, amino acid sequence, post-transitional modification, or biological activity of the expressed polypeptide; a pattern of expression that differs from wild type in terms of the effect of an environmental stimulus or extracellular stimulus on expression of the gene, *e.g*., a pattern of increased or decreased expression (as compared with wild type) in the presence of an increase or decrease in the strength of the stimulus.

"Subject", as used herein, can refer to a mammal, *e.g*., a human, or to an experimental or animal or disease model. The subject can also be a non-human animal, *e.g*., a horse, cow, goat, or other domestic animal.

A "purified preparation of cells", as used herein, refers to, in the case of plant or animal cells, an *in vitro* preparation of cells and not an entire intact plant or animal. In the case of cultured cells or microbial cells, it consists of a preparation of at least 10% and more preferably 50% of the subject cells.

Various aspects of the invention are described in further detail below.

### Isolated Nucleic Acid Molecules

Described herein is an isolated or purified, nucleic acid molecule that encodes a 15603 polypeptide, *e.g*., a full length 15603 protein or a fragment thereof, *e.g*., a biologically active portion of 15603 protein. Also described is a nucleic acid fragment suitable for use as a hybridization probe, which can be used, *e.g*., to identify a nucleic acid molecule encoding a 15603 polypeptide, 15603 mRNA, and fragments suitable for use as primers, *e.g*., PCR primers for the amplification or mutation of nucleic acid molecules.

Preferably, an isolated 15603 nucleic acid molecule includes the nucleotide sequence shown in SEQ ID NO:1, or a portion of any of this nucleotide sequence. Preferably, the nucleic acid molecule includes sequences encoding the human 15603 protein (i.e., "the coding region" of SEQ ID NO:1, as shown in SEQ ID NO:3). Alternatively, the nucleic acid molecule can include only the coding region of SEQ ID NO:1 (*e.g.,* SEQ ID NO: 3) and, *e.g*., no flanking sequences which normally accompany the subject sequence. Alternatively, the nucleic acid molecule encodes a sequence corresponding to a fragment of the protein from about amino acid 493 to 727 or 123 to 347 of SEQ ID NO:2.

Alternatively, an isolated 15603 nucleic acid molecule includes a nucleic acid molecule which is a complement of the nucleotide sequence shown in SEQ ID NO:1 or SEQ ID NO:3, or a portion of any of these nucleotide sequences. Alternatively, the nucleic acid molecule is sufficiently complementary to the nucleotide sequence shown in SEQ ID NO:1 or SEQ ID NO:3 such that it can hybridize to the nucleotide sequence shown in SEQ ID NO:1 or 3, thereby forming a stable duplex.

Alternatively, an isolated 15603 nucleic acid molecule includes a nucleotide sequence which is at least about 70%, 75%, 80%, 85%, 90%, 91%, 92%, 93%, 94%, 95%, 96%, 97%, 98%, 99%, or more homologous to the entire length of the nucleotide sequence shown in SEQ NO:1 or SEQ ID NO:3, or a portion, preferably of the same length, of any of these nucleotide sequences.

### 15603 Nucleic Acid Fragments

A 15603 nucleic acid molecule can include only a portion of the nucleic acid sequence of SEQ ID NO:1 or 3. For example, such a nucleic acid molecule can include a fragment which can be used as a probe or primer or a fragment encoding a portion of a 15603 protein, *e.g*., an immunogenic or biologically active portion of a 15603 protein. A fragment can comprise those nucleotides of SEQ ID NO:1, which encode an ion channel protein domain of human 15603. The nucleotide sequence determined from the cloning of the 15603 gene allows for the generation of probes and primers designed for use in identifying and/or cloning other 15603 family members, or fragments thereof, as well as 15603 homologs, or fragments thereof, from other species.

Alternatively, a nucleic acid includes a nucleotide sequence that includes part, or all, of the coding region and extends into either (or both) the 5' or 3' noncoding region. Other alternatives include a fragment which includes a nucleotide sequence encoding an amino acid fragment described herein. Nucleic acid fragments can encode a specific domain or site described herein or fragments thereof, particularly fragments thereof which are at least 100, 200, or 300 amino acids in length. Fragments also include nucleic acid sequences corresponding to specific amino acid sequences described above or fragments thereof. Nucleic acid fragments should not to be construed as encompassing those fragments that may have been disclosed prior to the invention.

A nucleic acid fragment can include a sequence corresponding to a domain, region, or functional site described herein. A nucleic acid fragment can also include one or more domain, region, or functional site described herein. Thus, for example, a 15603 nucleic acid fragment can include a sequence corresponding to an ion channel protein domain, as described herein.

15603 probes and primers are disclosed. Typically a probe/primer is an isolated or purified oligonucleotide. The oligonucleotide typically includes a region of nucleotide sequence that hybridizes under stringent conditions to at least about 7, 12 or 15, preferably about 20 or 25, more preferably about 30, 35, 40, 45, 50, 55, 60, 65, or 75 consecutive nucleotides of a sense or antisense sequence of SEQ ID NO:1 or SEQ ID NO:3, or of a naturally occurring allelic variant or mutant of SEQ ID NO:1 or SEQ ID NO:3.

In a preferred embodiment the nucleic acid is a probe which is at least 5 or 10, and less than 200, more preferably less than 100, or less than 50, base pairs in length. It should be identical, or differ by 1, or less than in 5 or 10 bases, from a sequence disclosed herein. If alignment is needed for this comparison the sequences should be aligned for maximum homology. "Looped" out sequences from deletions or insertions, or mismatches, are considered differences.

A probe or primer can be derived from the sense or anti-sense strand of a nucleic acid which encodes: transmembrane domains of 15603, which are located at about amino acids 407 to 426, 443 to 459, 490 to 507, 598 to 614, 637 to 653, and 703 to 727 of SEQ ID NO:2 (predicted by MEMSAT, Jones et al. (1994) Biochemistry 33:3038-3049); or from the Ank repeat domain at amino acids 97 to 131, 132 to 163, and 218 to 250 of SEQ ID NO:2.

Furthermore a set of primers is described, *e.g*., primers suitable for use in a PCR, which can be used to amplify a selected region of a 15603 sequence, *e.g*., a domain, region, site or other sequence described herein. The primers should be at least 5, 10, or 50 base pairs in length and less than 100, or less than 200, base pairs in length. The primers should be identical, or differ by one base from a sequence disclosed herein or from a naturally occurring variant.

A nucleic acid fragment can encode an epitope bearing region of a polypeptide described herein.

A nucleic acid fragment encoding a "biologically active portion of a 15603 polypeptide" can be prepared by isolating a portion of the nucleotide sequence of SEQ ID NO:1 or 3, which encodes a polypeptide having a 15603 biological activity (*e.g*., the biological activities of the 15603 proteins are described herein), expressing the encoded portion of the 15603 protein (*e.g*., by recombinant expression *in vitro*) and assessing the activity of the encoded portion of the 15603 protein. For example, a nucleic acid fragment encoding a biologically active portion of 15603 includes ion channel domain, *e.g*., amino acid residues about 493 to 727 of SEQ ID NO:2. A nucleic acid fragment encoding a biologically active portion of a 15603 polypeptide, can comprise a nucleotide sequence which is greater than 200, 250, 300, or more nucleotides in length.

Preferably, a nucleic acid includes a nucleotide sequence which is about 300, 400, 500, 600, 700, 800, 900, 1000, 1100, 1200, 1300, 1400, 1500, 1600, 1700, 1800, 1900, 2100, 2200, 2300, 2400, 2500, 2600, 2700, or more nucleotides in length and hybridizes under stringent hybridization conditions to a nucleic acid molecule of SEQ ID NO:1 or SEQ ID NO:3.

### 15603 Nucleic Acid Variants

Also described are nucleic acid molecules that differ from the nucleic acid molecules that differ from the nucleotide sequence shown in SEQ ID NO:1 or SEQ ID NO:3. Such differences can be due to degeneracy of the genetic code (and result in a nucleic acid which encodes the same 15603 proteins as those encoded by the nucleotide sequence disclosed herein. Alternatively, an isolated 15603 nucleic acid molecule has a nucleotide sequence encoding a protein having an amino acid sequence which differs, by at least 1, but less than 5, 10, 20, 50, or 100 amino acid residues that shown in SEQ ID NO:2. If alignment is needed for this comparison the sequences should be aligned for maximum homology. "Looped" out sequences from deletions or insertions, or mismatches, are considered differences.

Nucleic acids can be chosen for having codons, which are preferred, or non-preferred, for a particular expression system. E.g., the nucleic acid can be one in which at least one codon, at preferably at least 10%, or 20% of the codons has been altered such that the sequence is optimized for expression in E. *coli,* yeast, human, insect, or CHO cells.

Nucleic acid variants can be naturally occurring, such as allelic variants (same locus), homologs (different locus), and orthologs (different organism) or can be non naturally occurring. Non-naturally occurring variants can be made by mutagenesis techniques, including those applied to polynucleotides, cells, or organisms. The variants can contain nucleotide substitutions, deletions, inversions and insertions. Variation can occur in either or both the coding and non-coding regions. The variations can produce both conservative and non-conservative amino acid substitutions (as compared in the encoded product).

Preferably, the nucleic acid differs from that of SEQ ID NO: 1 or 3, *e.g*., as follows: by at least one but less than 10, 20, 30, or 40 nucleotides; at least one but less than 1%, 5%, 10% or 20% of the nucleotides in the subject nucleic acid. If necessary for this analysis the sequences should be aligned for maximum homology. "Looped" out sequences from deletions or insertions, or mismatches, are considered differences.

Orthologs, homologs, and allelic variants can be identified using methods known in the art. These variants comprise a nucleotide sequence encoding a polypeptide that is 50%, at least about 55%, typically at least about 70-75%, more typically at least about 80-85%, and most typically at least about 90-95% or more identical to the nucleotide sequence shown in SEQ ID NO:2 or a fragment of this sequence. Such nucleic acid molecules can readily be identified as being able to hybridize under stringent conditions, to the nucleotide sequence shown in SEQ ID NO 2 or a fragment of the sequence. Nucleic acid molecules corresponding to orthologs, homologs, and allelic variants of the 15603 cDNAs of the invention can further be isolated by mapping to the same chromosome or locus as the 15603 gene.

Preferred variants include those that are correlated with the ability to regulate the flow of cation through a membrane by the presence of a second messenger such as diacylglycerol.

Allelic variants of 15603, *e.g*., human 15603, include both functional and non-functional proteins. Functional allelic variants are naturally occurring amino acid sequence variants of the 15603 protein within a population that maintain the ability to regulate diacyclglycerol activated second-messenger-operated cation channels. Functional allelic variants will typically contain only conservative substitution of one or more amino acids of SEQ ID NO:2, or substitution, deletion or insertion of non-critical residues in non-critical regions of the protein. Non-functional allelic variants are naturally-occurring amino acid sequence variants of the 15603, *e.g*., human 15603, protein within a population that do not have the ability to regulate diacyclglycerol activated second-messenger-operated cation channels. Non-functional allelic variants will typically contain a non-conservative substitution, a deletion, or insertion, or premature truncation of the amino acid sequence of SEQ ID NO:2, or a substitution, insertion, or deletion in critical residues or critical regions of the protein.

Moreover, nucleic acid molecules encoding other 15603 family members and, thus, which have a nucleotide sequence which differs from the 15603 sequences of SEQ ID NO:1 or SEQ ID NO:3 are also described.

### Antisense Nucleic Acid Molecules, Ribozymes and Modified 15603 Nucleic Acid Molecules

Also described herein is an isolated nucleic acid molecule which is antisense to 15603. An "antisense" nucleic acid can include a nucleotide sequence which is complementary to a "sense" nucleic acid encoding a protein, *e.g*., complementary to the coding strand of a double-stranded cDNA molecule or complementary to an mRNA sequence. The antisense nucleic acid can be complementary to an entire 15603 coding strand, or to only a portion thereof (*e.g*., the coding region of human 15603 corresponding to SEQ ID NO:3). Alternatively the antisense nucleic acid molecule is antisense to a "noncoding region" of the coding strand of a nucleotide sequence encoding 15603 (*e.g*., the 5' and 3' untranslated regions).

An antisense nucleic acid can be designed such that it is complementary to the entire coding region of 15603 mRNA, but more preferably is an oligonucleotide which is antisense to only a portion of the coding or noncoding region of 15603 mRNA. For example, the antisense oligonucleotide can be complementary to the region surrounding the translation start site of 15603 mRNA, *e.g*., between the -10 and +10 regions of the target gene nucleotide sequence of interest. An antisense oligonucleotide can be, for example, about 7, 10, 15, 20, 25, 30, 35, 40, 45, 50, 55, 60, 65, 70, 75, 80, or more nucleotides in length.

An antisense nucleic acid can be constructed using chemical synthesis and enzymatic ligation reactions using procedures known in the art. For example, an antisense nucleic acid (*e.g*., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e.g*., phosphorothioate derivatives and acridine substituted nucleotides can be used. The antisense nucleic acid also can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules are typically administered to a subject (*e.g.,* by direct injection at a tissue site), or generated *in situ* such that they hybridize with or bind to cellular mRNA and/or genomic DNA encoding a 15603 protein to thereby inhibit expression of the protein, *e.g*., by inhibiting transcription and/or translation. Alternatively, antisense nucleic acid molecules can be modified to target selected cells and then administered systemically. For systemic administration, antisense molecules can be modified such that they specifically or selectively bind to receptors or antigens expressed on a selected cell surface, *e.g*., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III, promoter are preferred.

Alternatively, the antisense nucleic acid molecule is an α-anomeric nucleic acid molecule. An α-anomeric nucleic acid molecule forms specific double-stranded hybrids with complementary RNA in which, contrary to the usual β-units, the strands run parallel to each other (Gaultier et al. (1987) Nucleic Acids. Res. 15:6625-6641). The antisense nucleic acid molecule can also comprise a 2'-o-methylribonucleotide (Inoue et al. (1987) Nucleic Acids Res. 15:6131-6148) or a chimeric RNA-DNA analogue (Inoue et al. (1987) FEBS Lett. 215:327-330).

Alternatively, an antisense nucleic acid is a ribozyme. A ribozyme having specificity for a 15603-encoding nucleic acid can include one or more sequences complementary to the nucleotide sequence of a 15603 cDNA disclosed herein (i.e., SEQ ID NO:1 or SEQ ID NO:3), and a sequence having known catalytic sequence responsible for mRNA cleavage (see U.S. Pat. No. 5,093,246 or Haselhoff and Gerlach (1988) Nature 334:585-591). For example, a derivative of a *Tetrahymena* L-19 IVS RNA can be constructed in which the nucleotide sequence of the active site is complementary to the nucleotide sequence to be cleaved in a 15603-encoding mRNA. See, *e.g*., Cech et al. U.S. Patent No. 4,987,071; and Cech et al. U.S. Patent No. 5,116,742. Alternatively, 15603 mRNA can be used to select a catalytic RNA having a specific ribonuclease activity from a pool of RNA molecules. See, *e.g*., Bartel and Szostak (1993) Science 261:1411-1418.

15603 gene expression can be inhibited by targeting nucleotide sequences complementary to the regulatory region of the 15603 (*e.g.,* the 15603 promoter and/or enhancers) to form triple helical structures that prevent transcription of the 15603 gene in target cells. See generally, Helene (1991) Anticancer Drug Des. 6:569-84; Helene (1992) Ann. N.Y. Acad. Sci. 660:27-36; and Maher (1992) Bioassays 14:807-15. The potential sequences that can be targeted for triple helix formation can be increased by creating a so-called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

Also described herein are detectably labeled oligonucleotide primer and probe molecules. Typically, such labels are chemiluminescent, fluorescent, radioactive, or colorimetric.

A 15603 nucleic acid molecule can be modified at the base moiety, sugar moiety or phosphate backbone to improve, *e.g*., the stability, hybridization, or solubility of the molecule. For example, the deoxyribose phosphate backbone of the nucleic acid molecules can be modified to generate peptide nucleic acids (see Hyrup et al. (1996) Bioorganic & Medicinal Chemistry 4: 5-23). As used herein, the terms "peptide nucleic acid" or "PNA" refers to a nucleic acid mimic, *e.g*., a DNA mimic, in which the deoxyribose phosphate backbone is replaced by a pseudopeptide backbone and only the four natural nucleobases are retained. The neutral backbone of a PNA can allow for specific hybridization to DNA and RNA under conditions of low ionic strength. The synthesis of PNA oligomers can be performed using standard solid phase peptide synthesis protocols as described in Hyrup *et al.* (1996) *supra;* Perry-O'Keefe et al. (1996) Proc. Natl. Acad. Sci. 93: 14670-675.

PNAs of 15603 nucleic acid molecules can be used in therapeutic and diagnostic applications. For example, PNAs can be used as antisense or antigene agents for sequence-specific modulation of gene expression by, for example, inducing transcription or translation arrest or inhibiting replication. PNAs of 15603 nucleic acid molecules can also be used in the analysis of single base pair mutations in a gene, (*e.g*., by PNA-directed PCR clamping); as 'artificial restriction enzymes' when used in combination with other enzymes, (*e.g*., S1 nucleases (Hyrup *et al.* (1996) *supra*)); or as probes or primers for DNA sequencing or hybridization (Hyrup *et al.* (1996) *supra;* Perry-O'Keefe *supra*).

Alternatively, the oligonucleotide can include other appended groups such as peptides (*e.g*., for targeting host cell receptors *in vivo*), or agents facilitating transport across the cell membrane (see, *e.g*., Letsinger et al. (1989) Proc. Natl. Acad. Sci. USA 86:6553-6556; Lemaitre et al. (1987) Proc. Natl. Acad. Sci. USA 84:648-652; PCT Publication No. WO88/09810) or the blood-brain barrier (see, *e.g.,* PCT Publication No. WO89/10134). In addition, oligonucleotides can be modified with hybridization-triggered cleavage agents (see, *e.g*., Krol et al. (1988) Bio-Techniques 6:958-976) or intercalating agents. (see, *e.g*., Zon (1988) Pharm. Res. 5:539-549). To this end, the oligonucleotide can be conjugated to another molecule, (*e.g*., a peptide, hybridization triggered crosslinking agent, transport agent, or hybridization-triggered cleavage agent).

Also disclosed herein are molecular beacon oligonucleotide primer and probe molecules having at least one region which is complementary to a 15603 nucleic acid, two complementary regions one having a fluorophore and one a quencher such that the molecular beacon is useful for quantitating the presence of the 15603 nucleic acid of the invention in a sample. Molecular beacon nucleic acids are described, for example, in Lizardi et al., U.S. Patent No. 5,854,033; Nazarenko et al., U.S. Patent No. 5,866,336, and Livak et al., U.S. Patent 5,876,930.

### Isolated 15603 Polypeptides

Also described herein are an isolated 15603 protein, or fragment, *e.g*., a biologically active portion, for use as immunogens or antigens to raise or test (or more generally to bind) anti-15603 antibodies. 15603 protein can be isolated from cells or tissue sources using standard protein purification techniques. 15603 protein or fragments thereof can be produced by recombinant DNA techniques or synthesized chemically.

Polypeptides described herein include those which arise as a result of the existence of multiple genes, alternative transcription events, alternative RNA splicing events, and alternative translational and post-translational events. The polypeptide can be expressed in systems, *e.g*., cultured cells, which result in substantially the same post-translational modifications present when the polypeptide is expressed in a native cell, or in systems which result in the alteration or omission of post-translational modifications, *e.g*., glycosylation or cleavage, present in a native cell.

Preferably, a 15603 polypeptide has one or more of the following characteristics:

it has the ability to regulate the flow of cations through a membrane, ability to bind a second messenger, ability to bind diacylglycerol, ability to regulate intracellular calcium levels, and ability to regulate angiogenesis;

it has a molecular weight, *e.g*., a deduced molecular weight, preferably ignoring any contribution of post translational modifications, amino acid composition or other physical characteristic of a 15603 polypeptide, *e.g*., a polypeptide of SEQ ID NO:2;

it has an overall sequence similarity of at least 60%, preferably at least 70%, more preferably at least 80, 90, or 95%, with a polypeptide of SEQ ID NO:2;

it is expressed in at least the following human tissues and cell lines: at high levels in names of tissues list and taqman hemangioma, vascular smooth muscle, megakaryocytes, Wilm's tumor; at medium levels in normal brain cortex, uterine, and poliferative endothelial cells; and at low levels in adipose tissue, normal human internal mammary artery, diseased human aorta, diseased human artery, diseased human vein, and normal human vein, normal heart, CHF heart, normal kidney, skeletal muscle, pancreas, primary osteoblasts, brain hypothalamus, normal breast, normal ovary, normal prostate, prostate tumor, salivary glands, normal colon, colon tumor, normal lung, lung tumor, lung COPD, colon IBD, normal spleen, normal tonsil, normal small intestine, skin decubitus, synovium, glioblastomas, fetal adrenal, fetal kidney, and fetal heart.

it has an ion channel protein domain which is preferably about 70%, 80%, 90% or 95% identical to amino acid residues about 493 to 727 of SEQ ID NO:2;

it has an ank repeat domain which is preferably about 70%, 80%, 90% or 95% identical to amino acid residues about 97 to 131, 132 to 163, and 218 to 250 of SEQ ID NO:2; and

Preferably, the 15603 protein, or fragment thereof, differs from the corresponding sequence in SEQ ID NO:2. in one embodiment it differs by at least one but by less than 15, 10 or 5 amino acid residues. In another it differs from the corresponding sequence in SEQ ID NO:2 by at least one residue but less than 20%, 15%, 10% or 5% of the residues in it differ from the corresponding sequence in SEQ ID NO:2. (if this comparison requires alignment the sequences should be aligned for maximum homology. "looped" out sequences from deletions or insertions, or mismatches, are considered differences.) the differences are, preferably, differences or changes at a non-essential residue or a conservative substitution. Preferably, the differences are not in the ank repeat domain at about residues 97 to 131, 132 to 163, and 218 to 250 of SEQ ID NO:2. Alternatively one or more differences are in the ank repeat domain at about residues 97 to 131, 132 to 163, and 218 to 250 of SEQ ID NO:2.

Other alternatives include a protein that contains one or more changes in amino acid sequence, *e.g*., a change in an amino acid residue which is not essential for activity. Such 15603 proteins differ in amino acid sequence from SEQ ID NO:2, yet retain biological activity.

Alternatively, the protein includes an amino acid sequence at least about 60%, 65%, 70%, 75%, 80%, 85%, 90%, 95%, 98% or more homologous to SEQ ID NO:2.

A 15603 protein or fragment is provided which varies from the sequence of SEQ ID NO:2 in regions defined by amino acids about residues 407 to 426, 443 to 459, 490 to 507, 598 to 614, 637 to 653, or 703 to 727 of SEQ ID NO:2 by at least one but by less than 15, 10 or 5 amino acid residues in the protein or fragment. In some alternatives the difference is at a non-essential residue or is a conservative substitution, while in others the difference is at an essential residue or is a non-conservative substitution.

A 15603 protein or fragment is provided which varies from the sequence of SEQ ID NO:2 in regions defined by amino acids about residues 97 to 131, 132 to 163, and 218 to 250 of SEQ ID NO:2 by at least one but by less than 15, 10 or 5 amino acid residues in the protein or fragment. In some alternatives the difference is at a non-essential residue or is a conservative substitution, while in others the difference is at an essential residue or is a non-conservative substitution.

In one alternative, a biologically active portion of a 15603 protein includes at least one transmembrane domain. Moreover, other biologically active portions, in which other regions of the protein are deleted, can be prepared by recombinant techniques and evaluated for one or more of the functional activities of a native 15603 protein.

Preferably, the 15603 protein has an amino acid sequence shown in SEQ ID NO:2. In other embodiments, the 15603 protein is sufficiently or substantially identical to SEQ ID NO:2. Alternatively, the 15603 protein is sufficiently or substantially identical to SEQ ID NO:2 and retains the functional activity of the protein of SEQ ID NO:2, as described in detail in the subsections above.

### 15603 Chimeric or Fusion Proteins

Also disclosed herein are 15603 chimeric or fusion proteins. As used herein, a 15603 "chimeric protein" or "fusion protein" includes a 15603 polypeptide linked to a non-15603 polypeptide. A "non-15603 polypeptide" refers to a polypeptide having an amino acid sequence corresponding to a protein which is not substantially homologous to the 15603 protein, *e.g*., a protein which is different from the 15603 protein and which is derived from the same or a different organism. The 15603 polypeptide of the fusion protein can correspond to all or a portion *e.g*., a fragment described herein of a 15603 amino acid sequence. Preferably, a 15603 fusion protein includes at least one (or two) biologically active portion of a 15603 protein. The non-15603 polypeptide can be fused to the N-terminus or C-terminus of the 15603 polypeptide.

The fusion protein can include a moiety which has a high affinity for a ligand. For example, the fusion protein can be a GST-15603 fusion protein in which the 15603 sequences are fused to the C-terminus of the GST sequences. Such fusion proteins can facilitate the purification of recombinant 15603. Alternatively, the fusion protein can be a 15603 protein containing a heterologous signal sequence at its N-terminus. In certain host cells (*e.g*., mammalian host cells), expression and/or secretion of 15603 can be increased through use of a heterologous signal sequence.

Fusion proteins can include all or a part of a serum protein, *e.g*., a portion of an immunoglobulin (*e.g*., IgG, IgA, or IgE), *e.g.,* an Fc region and/or the hinge C1 and C2 sequences of an immunoglobulin or human serum albumin.

The 15603 fusion proteins can be incorporated into pharmaceutical compositions and administered to a subject *in vivo.* The 15603 fusion proteins can be used to affect the bioavailability of a 15603 substrate. 15603 fusion proteins can be useful therapeutically for the treatment of disorders caused by, for example, (i) aberrant modification or mutation of a gene encoding a 15603 protein; (ii) mis-regulation of the 15603 gene; and (iii) aberrant post-translational modification of a 15603 protein.

Moreover, the 15603-fusion proteins can be used as immunogens to produce anti-15603 antibodies in a subject, to purify 15603 ligands and in screening assays to identify molecules which inhibit the interaction of 15603 with a 15603 substrate.

Expression vectors are commercially available that already encode a fusion moiety (*e.g*., a GST polypeptide). A 15603-encoding nucleic acid can be cloned into such an expression vector such that the fusion moiety is linked in-frame to the 15603 protein.

### Variants of 15603 Proteins

Also disclosed herein is a variant of a 15603 polypeptide, *e.g*., which functions as an agonist (mimetics) or as an antagonist. Variants of the 15603 proteins can be generated by mutagenesis, *e.g*., discrete point mutation, the insertion or deletion of sequences or the truncation of a 15603 protein. An agonist of the 15603 proteins can retain substantially the same, or a subset, of the biological activities of the naturally occurring form of a 15603 protein. An antagonist of a 15603 protein can inhibit one or more of the activities of the naturally occurring form of the 15603 protein by, for example, competitively modulating a 15603-mediated activity of a 15603 protein. Thus, specific biological effects can be elicited by treatment with a variant of limited function. Preferably, treatment of a subject with a variant having a subset of the biological activities of the naturally occurring form of the protein has fewer side effects in a subject relative to treatment with the naturally occurring form of the 15603 protein.

Variants of a 15603 protein can be identified by screening combinatorial libraries of mutants, *e.g*., truncation mutants, of a 15603 protein for agonist or antagonist activity.

Libraries of fragments *e.g*., N terminal, C terminal, or internal fragments, of a 15603 protein coding sequence can be used to generate a variegated population of fragments for screening and subsequent selection of variants of a 15603 protein.

Variants in which a cysteine residues is added or deleted or in which a residue which is glycosylated is added or deleted are particularly preferred.

Methods for screening gene products of combinatorial libraries made by point mutations or truncation, and for screening cDNA libraries for gene products having a selected property are known in the art. Recursive ensemble mutagenesis (REM), a new technique which enhances the frequency of functional mutants in the libraries, can be used in combination with the screening assays to identify 15603 variants (Arkin and Yourvan (1992) Proc. Natl. Acad. Sci. USA 89:7811-7815; Delgrave et al. (1993) Protein Engineering 6:327-331).

Cell based assays can be exploited to analyze a variegated 15603 library. For example, a library of expression vectors can be transfected into a cell line, *e.g*., a cell line, which ordinarily responds to 15603 in a substrate-dependent manner. The transfected cells are then contacted with 15603 and the effect of the expression of the mutant on signaling by the 15603 substrate can be detected, *e.g*., by measuring membrane potentials or diacylglycerol binding. Plasmid DNA can then be recovered from the cells which score for inhibition, or alternatively, potentiation of signaling by the 15603 substrate, and the individual clones further characterized.

Also described herein is a method of making a 15603 polypeptide, *e.g.,* a peptide having a non-wild type activity, *e.g*., an antagonist, agonist, or super agonist of a naturally occurring 15603 polypeptide, *e.g*., a naturally occurring 15603 polypeptide. The method includes altering the sequence of a 15603 polypeptide, *e.g*., altering the sequence, *e.g*., by substitution or deletion of one or more residues of a non-conserved region, a domain or residue disclosed herein, and testing the altered polypeptide for the desired activity.

Also described herein is a method of making a fragment or analog of a 15603 polypeptide a biological activity of a naturally occurring 15603 polypeptide. The method includes altering the sequence, *e.g*., by substitution or deletion of one or more residues, of a 15603 polypeptide, *e.g*., altering the sequence of a non-conserved region, or a domain or residue described herein, and testing the altered polypeptide for the desired activity.

### Anti-15603 Antibodies

Also described herein is an anti-15603 antibody. The term "antibody" as used herein refers to an immunoglobulin molecule or immunologically active portion thereof, i.e., an antigen-binding portion. Examples of immunologically active portions of immunoglobulin molecules include scFV and dcFV fragments, Fab and F(ab')₂ fragments which can be generated by treating the antibody with an enzyme such as papain or pepsin, respectively.

The antibody can be a polyclonal, monoclonal, recombinant, *e.g*., a chimeric or humanized, fully human, non-human, *e.g*., murine, or single chain antibody. In a preferred embodiment it has effector function and can fix complement. The antibody can be coupled to a toxin or imaging agent.

A full-length 15603 protein or, autogenic peptide fragment of 15603 can be used as an immunogen or can be used to identify anti-15603 antibodies made with other immunogens, *e.g*., cells, membrane preparations, and the like. The antigenic peptide of 15603 should include at least 8 amino acid residues of the amino acid sequence shown in SEQ ID NO:2 and encompasses an epitope of 15603. Preferably, the antigenic peptide includes at least 10 amino acid residues, more preferably at least 15 amino acid residues, even more preferably at least 20 amino acid residues, and most preferably at least 30 amino acid residues.

### Recombinant Expression vectors Host Cells and Genetically Engineered Cells

Also described herein are vectors, preferably expression vectors, containing a nucleic acid encoding a polypeptide described herein. As used herein, the term "vector" refers to a nucleic acid molecule capable of transporting another nucleic acid to which it has been linked and can include a plasmid, cosmid or viral vector. The vector can be capable of autonomous replication or it can integrate into a host DNA. Viral vectors include, *e.g*., replication defective retroviruses, adenoviruses and adeno-associated viruses.

A vector can include a 15603 nucleic acid in a form suitable for expression of the nucleic acid in a host cell. Preferably the recombinant expression vector includes one or more regulatory sequences operatively linked to the nucleic acid sequence to be expressed. The term "regulatory sequence" includes promoters, enhancers and other expression control elements (*e.g.*, polyadenylation signals). Regulatory sequences include those which direct constitutive expression of a nucleotide sequence, as well as tissue-specific regulatory and/or inducible sequences. The design of the expression vector can depend on such factors as the choice of the host cell to be transformed, the level of expression of protein desired, and the like. The expression vectors can be introduced into host cells to thereby produce proteins or polypeptides, including fusion proteins or polypeptides, encoded by nucleic acids as described herein (*e.g*., 15603 proteins, mutant forms of 15603 proteins, fusion proteins, and the like).

The recombinant expression vectors can be designed for expression of 15603 proteins in prokaryotic or eukaryotic cells. For example, polypeptides of the invention can be expressed in *E. coli,* insect cells (*e.g*., using baculovirus expression vectors), yeast cells or mammalian cells. Suitable host cells are discussed further in Goeddel, (1990) Gene Expression Technology: Methods in Enzymology 1885, Academic Press, San Diego, CA. Alternatively, the recombinant expression vector can be transcribed and translated *in vitro,* for example using T7 promoter regulatory sequences and T7 polymerase.

Expression of proteins in prokaryotes is most often carried out in E. *coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson (1988) Gene 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Purified fusion proteins can be used in 15603 activity assays, (*e.g*., direct assays or competitive assays described in detail below), or to generate antibodies specific or selective for 15603 proteins. Preferably, a fusion protein expressed in a retroviral expression vector can be used to infect bone marrow cells which are subsequently transplanted into irradiated recipients. The pathology of the subject recipient is then examined after sufficient time has passed (*e.g*., six weeks).

To maximize recombinant protein expression in E. *coli* is to express the protein in a host bacteria with an impaired capacity to proteolytically cleave the recombinant protein (Gottesman (1990) Gene Expression Technology: Methods in Enzymology 185, Academic Press, San Diego, California 119-128). Another strategy is to alter the nucleic acid sequence of the nucleic acid to be inserted into an expression vector so that the individual codons for each amino acid are those preferentially utilized in *E. coli* (Wada et al., (1992) Nucleic Acids Res. 20:2111-2118). Such alteration of nucleic acid sequences of the invention can be carried out by standard DNA synthesis techniques.

The 15603 expression vector can be a yeast expression vector, a vector for expression in insect cells, *e.g*., a baculovirus expression vector or a vector suitable for expression in mammalian cells.

When used in mammalian cells, the expression vector's control functions are often provided by viral regulatory elements. For example, commonly used promoters are derived from polyoma, Adenovirus 2, cytomegalovirus and Simian Virus 40.

Alternatively, the recombinant mammalian expression vector is capable of directing expression of the nucleic acid preferentially in a particular cell type (*e.g*., tissue-specific regulatory elements are used to express the nucleic acid). Nonlimiting examples of suitable tissue-specific promoters include the albumin promoter (liver-specific; Pinkert et al. (1987) Genes Dev. 1:265-277), lymphoid-specific promoters (Calame and Eaton (1988) Adv. Immunol. 43:235-275), in particular promoters of T cell receptors (Winoto and Baltimore (1989) EMBO J. 8:729-733) and immunoglobulins (Banerji et al. (1983) Cell 33:729-740; Queen and Baltimore (1983) Cell 33:741-748), neuron-specific promoters (*e.g*., the neurofilament promoter; Byrne and Ruddle (1989) Proc. Natl. Acad. Sci. USA 86:5473-5477), pancreas-specific promoters (Edlund et al. (1985) Science 230:912-916), and mammary gland-specific promoters (*e.g*., milk whey promoter; U.S. Patent No. 4,873,316 and European Application Publication No. 264,166). Developmentally-regulated promoters are also encompassed, for example, the murine hox promoters (Kessel and Gruss (1990) Science 249:374-379) and the α-fetoprotein promoter (Campes and Tilghman (1989) Genes Dev. 3:537-546).

Also described is a recombinant expression vector comprising a DNA molecule of the invention cloned into the expression vector in an antisense orientation. Regulatory sequences (*e.g*., viral promoters and/or enhancers) operatively linked to a nucleic acid cloned in the antisense orientation can be chosen which direct the constitutive, tissue specific or cell type specific expression of antisense RNA in a variety of cell types. The antisense expression vector can be in the form of a recombinant plasmid, phagemid or attenuated virus. For a discussion of the regulation of gene expression using antisense genes see Weintraub et al., (1986) Reviews - Trends in Genetics 1:1.

Also described herein is a host cell which includes a nucleic acid molecule described herein, *e.g*., a 15603 nucleic acid molecule within a recombinant expression vector or a 15603 nucleic acid molecule containing sequences which allow it to homologously recombine into a specific site of the host cell's genome. The terms "host cell" and "recombinant host cell" are used interchangeably herein. Such terms refer not only to the particular subject cell but to the progeny or potential progeny of such a cell. Because certain modifications can occur in succeeding generations due to either mutation or environmental influences, such progeny may not, in fact, be identical to the parent cell, but are still included within the scope of the term as used herein.

A host cell can be any prokaryotic or eukaryotic cell. For example, a 15603 protein can be expressed in bacterial cells such as E. *coli,* insect cells, yeast or mammalian cells (such as Chinese hamster ovary (CHO) cells or CV-1 origin, SV-40 (COS) cells). Other suitable host cells are known to those skilled in the art.

Vector DNA can be introduced into host cells via conventional transformation or transfection techniques. As used herein, the terms "transformation" and "transfection" are intended to refer to a variety of art-recognized techniques for introducing foreign nucleic acid (*e.g*., DNA) into a host cell, including calcium phosphate or calcium chloride co-precipitation, DEAE-dextran-mediated transfection, lipofection, or electroporation.

A host cell can be used to produce (i.e., express) a 15603 protein. Accordingly, the invention further provides methods for producing a 15603 protein using the host cells of the invention. For example, the method includes culturing the host cell of the invention (into which a recombinant expression vector encoding a 15603 protein has been introduced) in a suitable medium such that a 15603 protein is produced. Alternatively, the method further includes isolating a 15603 protein from the medium or the host cell.

Also disclosed is a cell or purified preparation of cells which include a 15603 transgene, or which otherwise misexpress 15603. The cell preparation can consist of human or non-human cells, *e.g*., rodent cells, *e.g*., mouse or rat cells, rabbit cells, or pig cells. Preferably, the cell or cells include a 15603 transgene, *e.g*., a heterologous form of a 15603, *e.g.,* a gene derived from humans (in the case of a non-human cell). The 15603 transgene can be misexpressed, *e.g*., overexpressed or underexpressed. Preferably, the cell or cells include a gene which misexpresses an endogenous 15603, *e.g.,* a gene the expression of which is disrupted, *e.g*., a knockout. Such cells can serve as a model for studying disorders which are related to mutated or misexpressed 15603 alleles or for use in drug screening.

Also described is a human cell, *e.g*., a hematopoietic stem cell, transformed with nucleic acid which encodes a subject 15603 polypeptide.

Also described are cells, preferably human cells, *e.g*., human hematopoietic or fibroblast cells, in which an endogenous 15603 is under the control of a regulatory sequence that does not normally control the expression of the endogenous 15603 gene. The expression characteristics of an endogenous gene within a cell, *e.g.,* a cell line or microorganism, can be modified by inserting a heterologous DNA regulatory element into the genome of the cell such that the inserted regulatory element is operably linked to the endogenous 15603 gene. For example, an endogenous 15603 gene which is "transcriptionally silent," *e.g.*, not normally expressed, or expressed only at very low levels, can be activated by inserting a regulatory element which is capable of promoting the expression of a normally expressed gene product in that cell. Techniques such as targeted homologous recombinations, can be used to insert the heterologous DNA as described in, *e.g.*, Chappel, US 5,272,071; WHO 91/06667, published in May 16,1991.

### Uses

The nucleic acid molecules, proteins, protein homologs, and antibodies described herein can be used in one or more of the following methods: a) screening assays;

The isolated nucleic acid molecules described herein can be used, for example, to express a 15603 protein *(e*.*g*., via a recombinant expression vector in a host cell in gene therapy applications), to detect a 15603 mRNA. (*e.g.*, in a biological sample) or a genetic alteration in a 15603 gene, and to modulate 15603 activity, as described further below. The 15603 proteins can be used to treat disorders characterized by insufficient or excessive production of a 15603 substrate or production of 15603 inhibitors. In addition, the 15603 proteins can be used to screen for naturally occurring 15603 substrates, to screen for drugs or compounds which modulate 15603 activity, as well as to treat disorders characterized by insufficient or excessive production of 15603 protein or production of 15603 protein forms which have decreased, aberrant or unwanted activity compared to 15603 wild type protein (*e.g.*, aberrant or deficient ion channel function or expression). Moreover, the anti-15603 antibodies of the invention can be used to detect and isolate 15603 proteins, regulate the bioavailability of 15603 proteins, and modulate 15603 activity.

A method of evaluating a compound for the ability to interact with, *e.g.*, bind, a subject 15603 polypeptide is provided. The method includes: contacting the compound with the subject 15603 polypeptide; and evaluating ability of the compound to interact with, *e.g.*, to bind or form a complex with the subject 15603 polypeptide. This method can be performed *in vitro, e*.*g*., in a cell free system, or in *vivo*, *e*.*g*., in a two-hybrid interaction trap assay. This method can be used to identify naturally occurring molecules which interact with subject 15603 polypeptide. It can also be used to find natural or synthetic inhibitors of subject 15603 polypeptide. Screening methods are discussed in more detail below.

### Screening Assays:

The invention provides methods (also referred to herein as "screening assays") for identifying modulators, i.e., candidate or test compounds or agents (*e.g.*, proteins, peptides, peptidomimetics, peptoids, small molecules or other drugs) which have an inhibitory effect on 15603 expression or 15603 activity. Compounds thus identified can be used to modulate the activity of target gene products (*e.g.*, 15603 genes) in a therapeutic protocol, to elaborate the biological function of the target gene product, or to identify compounds that disrupt normal target gene interactions.

Also described are assays for screening candidate or test compounds which are substrates of a 15603 protein or polypeptide or a biologically active portion thereof. Also described are assays for screening candidate or test compounds which bind to or modulate the activity of a 15603 protein or polypeptide or a biologically active portion thereof.

The test compounds used in the present invention can be obtained using any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive; see, *e.g.*, Zuckermann et al. (1994) J. Med. Chem. 37:2678-85); spatially addressable parallel solid phase or solution phase libraries; synthetic library methods requiring deconvolution; the 'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds (Lam (1997) Anticancer Drug Des.12:145).

Examples of methods for the synthesis of molecular libraries can be found in the art, for example in: DeWitt et al. (1993) Proc. Natl. Acad. Sci. U.S.A. 90:6909-13; Erb et al. (1994) Proc. Natl. Acad. Sci. USA 91:11422-426; Zuckermann et al. (1994). J. Med. Chem. 37:2678-85; Cho et al. (1993) Science 261:1303; Carrell et al. (1994) Angew. Chem. Int. Ed. Engl. 33:2059; Carell et al. (1994) Angew. Chem. Int. Ed. Engl. 33:2061; and in Gallop et al. (1994) J. Med. Chem. 37:1233-51.

Libraries of compounds can be presented in solution (*e.g.*, Houghten (1992) Biotechniques 13:412-421), or on beads (Lam (1991) Nature 354:82-84), chips (Fodor (1993) Nature 364:555-556), bacteria (Ladner, USP 5,223,409), spores (Ladner USP '409), plasmids (Cull et al. (1992) Proc Natl Acad Sci USA 89:1865-1869) or on phage (Scott and Smith (1990) Science 249:386-390; Devlin (1990) Science 249:404-406; Cwirla et al. (1990) Proc. Natl. Acad. Sci. 87:6378-6382; Felici (1991) J. Mol. Biol. 222:301-310; Ladner *supra*.).

In one embodiment, an assay is a cell-based assay in which a cell which expresses a 15603 protein or biologically active portion thereof is contacted with a test compound, and the ability of the test compound to inhibit 15603 activity is determined. Determining the ability of the test compound to modulate 15603 activity can be accomplished by monitoring ion channel function. The cell, for example, can be of mammalian origin, *e.g.*, human.

The ability of the test compound to modulate 15603 binding to a compound, *e.g.*, a 15603 substrate, or to bind to 15603 can also be evaluated. This can be accomplished, for example, by coupling the compound, *e.g.*, the substrate, with a radioisotope or enzymatic label such that binding of the compound, *e.g.*, the substrate, to 15603 can be determined by detecting the labeled compound, *e.g.*, substrate, in a complex. Alternatively, 15603 could be coupled with a radioisotope or enzymatic label to monitor the ability of a test compound to modulate 15603 binding to a 15603 substrate in a complex. For example, compounds (*e.g.*, 15603 substrates) can be labeled with ¹²¹I, ¹⁴C, ³⁵S or ³H., either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

The ability of a compound (*e.g.*, a 15603 substrate) to interact with 15603 with or without the labeling of any of the interactants can be evaluated. For example, a microphysiometer can be used to detect the interaction of a compound with 15603 without the labeling of either the compound or the 15603. McConnell et al. (1992) Science 257:1906-1912. As used herein, a "microphysiometer" (*e.g.*, Cytosensor) is an analytical instrument that measures the rate at which a cell acidifies its environment using a light-addressable potentiometric sensor (LAPS). Changes in this acidification rate can be used as an indicator of the interaction between a compound and 15603.

In yet another embodiment, a cell-free assay is provided in which a 15603 protein is contacted with a test compound and the ability of the test compound to inhibit the 15603 protein is evaluated. Preferred biologically active portions of the 15603 proteins to be used in assays described herein include fragments which participate in interactions with non-15603 molecules, *e.g.*, fragments with high surface probability scores.

Soluble and/or membrane-bound forms of 15603 proteins can be used in the cell-free assays of the invention. When membrane-bound forms of the protein are used, it may be desirable to utilize a solubilizing agent. Examples of such solubilizing agents include non-ionic detergents such as n-octylglucoside, n-dodecylglucoside, n-dodecylmaltoside, octanoyl-N-methylglucamide, decanoyl-N-methylglucamide, Triton^{®} X-100, Triton^{®} X-114, Thesit^{®}, Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

Cell-free assays involve preparing an ion channel in a plasma membrane and measuring the flow of ions throught the pore of the membrane, e.g., by patch clamp recording. If binding of the ion channel or related subunit or protein and a test compound or protein is required for activation of the channel, this can be accomplished under conditions and for a time sufficient to allow the two components to interact and bind, thus forming a complex.

The interaction between two molecules can also be detected, *e.g.*, using fluorescence energy transfer (FET) (see, for example, Lakowicz et al., U.S. Patent No. 5,631,169; Stavrianopoulos, et al., U.S. Patent No. 4,868,103). A fluorophore label on the first, 'donor' molecule is selected such that its emitted fluorescent energy will be absorbed by a fluorescent label on a second, 'acceptor' molecule, which in turn is able to fluoresce due to the absorbed energy. Alternately, the 'donor' protein molecule can simply utilize the natural fluorescent energy of tryptophan residues. Labels are chosen that emit different wavelengths of light, such that the 'acceptor' molecule label can be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (*e.g.*, using a fluorimeter).

Alternatively, determining the ability of the 15603 protein to bind to a target molecule can be accomplished using real-time Biomolecular Interaction Analysis (BIA) (see, *e.g.*, Sjolander and Urbaniczky (1991) Anal. Chem. 63:2338-2345 and Szabo et al. (1995) Curr. Opin. Struct. Biol. 5:699-705). "Surface plasmon resonance" or "BIA" detects biospecific interactions in real time, without labeling any of the interactants (*e.g.*, BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

For example, the target gene product or the test substance is anchored onto a solid phase. The target gene product/test compound complexes anchored on the solid phase can be detected at the end of the reaction. Preferably, the target gene product can be anchored onto a solid surface, and the test compound, (which is not anchored), can be labeled, either directly or indirectly, with detectable labels discussed herein.

It may be desirable to immobilize either 15603, an anti-15603 antibody or its target molecule to facilitate separation of complexed from uncomplexed forms of one or both of the proteins, as well as to accommodate automation of the assay. Binding of a test compound to a 15603 protein, or interaction of a 15603 protein with a target molecule in the presence and absence of a candidate compound, can be accomplished in any vessel suitable for containing the reactants. Examples of such vessels include microtiter plates, test tubes, and micro-centrifuge tubes. For example, a fusion protein can be provided which adds a domain that allows one or both of the proteins to be bound to a matrix. For example, glutathione-S-transferase/15603 fusion proteins or glutathione-S-transferase/target fusion proteins can be adsorbed onto glutathione sepharose beads (Sigma Chemical, St. Louis, MO) or glutathione derivatized microtiter plates, which are then combined with the test compound or the test compound and either the non-adsorbed target protein or 15603 protein, and the mixture incubated under conditions conducive to complex formation (e.g., at physiological conditions for salt and pH). Following incubation, the beads or microtiter plate wells are washed to remove any unbound components, the matrix immobilized in the case of beads, complex determined either directly or indirectly, for example, as described above. Alternatively, the complexes can be dissociated from the matrix, and the level of 15603 binding or activity determined using standard techniques.

Other techniques for immobilizing either a 15603 protein or a target molecule on matrices include using conjugation of biotin and streptavidin. Biotinylated 15603 protein or target molecules can be prepared from biotin-NHS (N-hydroxy-succinimide) using techniques known in the art (*e.g.*, biotinylation kit, Pierce Chemicals, Rockford, IL), and immobilized in the wells of streptavidin-coated 96 well plates (Pierce Chemical).

In order to conduct the assay, the non-immobilized component is added to the coated surface containing the anchored component. After the reaction is complete, unreacted components are removed (*e.g.*, by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; *e.g.*, using a labeled antibody specific or selective for the immobilized component (the antibody, in turn, can be directly labeled or indirectly labeled with, *e.g.*, a labeled anti-Ig antibody).

For example, this assay is performed utilizing antibodies reactive with 15603 protein or target molecules but which do not interfere with binding of the 15603 protein to its target molecule. Such antibodies can be derivatized to the wells of the plate, and unbound target or 15603 protein trapped in the wells by antibody conjugation. Methods for detecting such complexes, in addition to those described above for the GST-immobilized complexes, include immunodetection of complexes using antibodies reactive with the 15603 protein or target molecule, as well as enzyme-linked assays which rely on detecting an enzymatic activity associated with the 15603 protein or target molecule.

Alternatively, cell free assays can be conducted in a liquid phase. In such an assay, the reaction products are separated from unreacted components, by any of a number of standard techniques, including but not limited to: differential centrifugation (see, for example, Rivas and Minton (1993) Trends Biochem Sci 18:284-7); chromatography (gel filtration chromatography, ion-exchange chromatography); electrophoresis (see, *e.g.,* Ausubel et al., eds. (1999) Current Protocols in Molecular Biology, J. Wiley, New York.); and immunoprecipitation (see, for example, Ausubel et al., eds. (1999) Current Protocols in Molecular Biology, J. Wiley, New York). Such resins and chromatographic techniques are known to one skilled in the art (see, *e.g.*, Heegaard (1998) J Mol Recognit 11:141-8*;* Hage and Tweed (1997) J Chromatogr B Biomed Sci Appl. 699:499-525). Further, fluorescence energy transfer can also be conveniently utilized, as described herein, to detect binding without further purification of the complex from solution.

Preferably, the assay includes contacting the 15603 protein or biologically active portion thereof with a known compound which binds 15603 to form an assay mixture, contacting the assay mixture with a test compound, and determining the ability of the test compound to interact with a 15603 protein, wherein determining the ability of the test compound to interact with a 15603 protein includes determining the ability of the test compound to preferentially bind to 15603 or biologically active portion thereof, or to modulate the activity of a target molecule, as compared to the known compound.

The target gene products described herein can, *in vivo,* interact with one or more cellular or extracellular macromolecules, such as proteins. For the purposes of this discussion, such cellular and extracellular macromolecules are referred to herein as "binding partners." Compounds that disrupt such interactions can be useful in regulating the activity of the target gene product. Such compounds can include, but are not limited to molecules such as antibodies, peptides, and small molecules. The preferred target genes/products are the 15603 genes herein identified. Also described herein are methods for determining the ability of the test compound to modulate the activity of a 15603 protein through modulation of the activity of a downstream effector of a 15603 target molecule. For example, the activity of the effector molecule on an appropriate target can be determined, or the binding of the effector to an appropriate target can be determined, as previously described.

To identify compounds that interfere with the interaction between the target gene product and its cellular or extracellular binding partner(s), a reaction mixture containing the target gene product and the binding partner is prepared, under conditions and for a time sufficient, to allow the two products to form complex. In order to test an inhibitory agent, the reaction mixture is provided in the presence and absence of the test compound. The test compound can be initially included in the reaction mixture, or can be added at a time subsequent to the addition of the target gene and its cellular or extracellular binding partner. Control reaction mixtures are incubated without the test compound or with a placebo. The formation of any complexes between the target gene product and the cellular or extracellular binding partner is then detected. The formation of a complex in the control reaction, but not in the reaction mixture containing the test compound, indicates that the compound interferes with the interaction of the target gene product and the interactive binding partner. Additionally, complex formation within reaction mixtures containing the test compound and normal target gene product can also be compared to complex formation within reaction mixtures containing the test compound and mutant target gene product. This comparison can be important in those cases wherein it is desirable to identify compounds that disrupt interactions of mutant but not normal target gene products.

These assays can be conducted in a heterogeneous or homogeneous format. Heterogeneous assays involve anchoring either the target gene product or the binding partner onto a solid phase, and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction between the target gene products and the binding partners, *e.g.*, by competition, can be identified by conducting the reaction in the presence of the test substance. Alternatively, test compounds that disrupt preformed complexes, *e.g.*, compounds with higher binding constants that displace one of the components from the complex, can be tested by adding the test compound to the reaction mixture after complexes have been formed. The various formats are briefly described below.

In a heterogeneous assay system, either the target gene product or the interactive cellular or extracellular binding partner, is anchored onto a solid surface (*e.g.*, a microtiter plate), while the non-anchored species is labeled, either directly or indirectly. The anchored species can be immobilized by non-covalent or covalent attachments. Alternatively, an immobilized antibody specific or selective for the species to be anchored can be used to anchor the species to the solid surface.

In order to conduct the assay, the partner of the immobilized species is exposed to the coated surface with or without the test compound. After the reaction is complete, unreacted components are removed (*e.g.*, by washing) and any complexes formed will remain immobilized on the solid surface. Where the non-immobilized species is pre-labeled, the detection of label immobilized on the surface indicates that complexes were formed. Where the non-immobilized species is not pre-labeled, an indirect label can be used to detect complexes anchored on the surface; *e.g.*, using a labeled antibody specific or selective for the initially non-immobilized species (the antibody, in turn, can be directly labeled or indirectly labeled with, *e.g.*, a labeled anti-Ig antibody). Depending upon the order of addition of reaction components, test compounds that inhibit complex formation or that disrupt preformed complexes can be detected.

Alternatively, the reaction can be conducted in a liquid phase in the presence or absence of the test compound, the reaction products separated from unreacted components, and complexes detected; *e.g.*, using an immobilized antibody specific or selective for one of the binding components to anchor any complexes formed in solution, and a labeled antibody specific or selective for the other partner to detect anchored complexes. Again, depending upon the order of addition of reactants to the liquid phase, test compounds that inhibit complex or that disrupt preformed complexes can be identified.

Alternatively, a homogeneous assay can be used. For example, a preformed complex of the target gene product and the interactive cellular or extracellular binding partner product is prepared in that either the target gene products or their binding partners are labeled, but the signal generated by the label is quenched due to complex formation (see, *e.g.*, U.S. Patent No. 4,109,496 that utilizes this approach for immunoassays). The addition of a test substance that competes with and displaces one of the species from the preformed complex will result in the generation of a signal above background. In this way, test substances that disrupt target gene product-binding partner interaction can be identified.

In yet another aspect, the 15603 proteins can be used as "bait proteins" in a two-hybrid assay or three-hybrid assay (see, e.g.*,* U.S. Patent No. 5,283,317; Zervos et al. (1993) Cell 72:223-232; Madura et al. (1993) J. Biol. Chem. 268:12046-12054; Bartel et al. (1993) Biotechniques 14:920-924; Iwabuchi et al. (1993) Oncogene 8:1693-1696; and Brent WO94/10300), to identify other proteins, which bind to or interact with 15603 ("15603-binding proteins" or "15603-bp") and are involved in 15603 activity. Such 15603-bps can be activators or inhibitors of signals by the 15603 proteins or 15603 targets as, for example, downstream elements of a 15603-mediated signaling pathway.

The two-hybrid system is based on the modular nature of most transcription factors, which consist of separable DNA-binding and activation domains. Briefly, the assay utilizes two different DNA constructs. In one construct, the gene that codes for a 15603 protein is fused to a gene encoding the DNA binding domain of a known transcription factor (*e.g.*, GAL-4). In the other construct, a DNA sequence, from a library of DNA sequences, that encodes an unidentified protein ("prey" or "sample") is fused to a gene that codes for the activation domain of the known transcription factor. (Alternatively the: 15603 protein can be the fused to the activator domain.) If the "bait" and the "prey" proteins are able to interact, *in vivo,* forming a 15603-dependent complex, the DNA-binding and activation domains of the transcription factor are brought into close proximity. This proximity allows transcription of a reporter gene (*e.g.*, lacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the 15603 protein.

Alternatively, modulators of 15603 expression are identified. For example, a cell or cell free mixture is contacted with a candidate compound and the expression of 15603 mRNA or protein evaluated relative to the level of expression of 15603 mRNA or protein in the absence of the candidate compound. When expression of 15603 mRNA or protein is greater in the presence of the candidate compound than in its absence, the candidate compound is identified as a stimulator of 15603 mRNA or protein expression. Alternatively, when expression of 15603 mRNA or protein is less (statistically significantly less) in the presence of the candidate compound than in its absence, the candidate compound is identified as an inhibitor of 15603 mRNA or protein expression. The level of 15603 mRNA or protein expression can be determined by methods described herein for detecting 15603 mRNA or protein.

Also described herein is a combination of two or more of the assays described herein. For example, a modulating agent can be identified using a cell-based or a cell free assay, and the ability of the agent to modulate the activity of a 15603 protein can be confirmed *in vivo, e*.*g*., in an animal such as an animal model for aberrant or deficient ion channel function or expression.

Also described herein are novel agents identified by the above-described screening assays. An agent identified as described herein (*e.g.*, a 15603 modulating agent, an antisense 15603 nucleic acid molecule, a 15603-specific antibody, or a 15603-binding partner) can be used in an appropriate animal model to determine the efficacy, toxicity, side effects, or mechanism of action, of treatment with such an agent. Furthermore, novel agents identified by the above-described screening assays can be used for treatments as described herein.

This invention is further illustrated by the following exemplification, which should not be construed as limiting.

### EXEMPLIFICATION

### Gene Expression Analysis

Total RNA was prepared from various human tissues by a single step extraction method using RNA STAT-60 according to the manufacturer instructions (TelTest, Inc). Each RNA preparation was treated with DNase I (Ambion) at 37°C for 1 hour. DNAse I treatment was determined to be complete if the sample required at least 38 PCR amplification cycles to reach a threshold level of fluorescence using β-2 microglobulin as an internal amplicon reference. The integrity of the RNA samples following DNase I treatment was confirmed by agarose gel electrophoresis and ethidium bromide staining. After phenol extraction cDNA was prepared from the sample using the SUPERSCRIPT™ Choice System following the manufacturer's instructions (GibcoBRL). A negative control of RNA without reverse transcriptase was mock reverse transcribed for each RNA sample.

Human 15603 expression was measured by TaqMan^{®} quantitative PCR (Perkin Elmer Applied Biosystems) in cDNA prepared from a variety of normal and diseased (e.g., cancerous) human tissues or cell lines.

Probes were designed by PrimerExpress software (PE Biosystems) based on the sequence of the human 15603 gene. Each human 15603 gene probe was labeled using FAM (6-carboxyfluorescein), and the β2-microglobulin reference probe was labeled with a different fluorescent dye, VIC. The differential labeling of the target gene and internal reference gene thus enabled measurement in same well. Forward and reverse primers and the probes for both β2-microglobulin and target gene were added to the TaqMan^{®} Universal PCR Master Mix (PE Applied Biosystems). Although the final concentration of primer and probe could vary, each was internally consistent within a given experiment. A typical experiment contained 200nM of forward and reverse primers plus 100nM probe for β-2 microglobulin and 600 nM forward and reverse primers plus 200 nM probe for the target gene. TaqMan matrix experiments were carried out on an ABI PRISM 7700 Sequence Detection System (PE Applied Biosystems). The thermal cycler conditions were as follows: hold for 2 min at 50°C and 10 min at 95°C, followed by two-step PCR for 40 cycles of 95°C for 15 sec followed by 60°C for 1 min.

The following method was used to quantitatively calculate human 15603 gene expression in the various tissues relative to β-2 microglobulin expression in the same tissue. The threshold cycle (Ct) value is defined as the cycle at which a statistically significant increase in fluorescence is detected. A lower Ct value is indicative of a higher mRNA concentration. The Ct value of the human 15603 gene is normalized by subtracting the Ct value of the β-2 microglobulin gene to obtain a _{Δ}Ct value using the following formula: ΔCt=Ct_{human 59914 and 59921} - Ct _{β-2 microglobulin}. Expression is then calibrated against a cDNA sample showing a comparatively low level of expression of the human 15603 gene. The _{Δ}Ct value for the calibrator sample is then subtracted from _{Δ}Ct for each tissue sample according to the following formula: _{ΔΔ}Ct=_{Δ}Ct-ₛₐₘₚₗₑ - _{Δ}Ct-_{calibrator}· Relative expression is then calculated using the arithmetic formula given by 2^{-ΔΔCt}. Expression of the target human 15603 gene in each of the tissues tested is then graphically represented as discussed in more detail below.

The results indicate significant 15603 expression in hemangioma, vascular smooth muscle, megakaryocytes, Wilm's tumor; at medium levels in normal brain cortex, uterine, and poliferative endothelial cells; and at low levels in adipose tissue, normal human internal mammary artery, diseased human aorta, diseased human artery, diseased human vein, and normal human vein, normal heart, CHF heart, normal kidney, skeletal muscle, pancreas, primary osteoblasts, brain hypothalamus, normal breast, normal ovary, normal prostate, prostate tumor, salivary glands, normal colon, colon tumor, normal lung, lung tumor, lung COPD, colon IBD, normal spleen, normal tonsil, normal small intestine, skin decubitus, synovium, glioblastomas, fetal adrenal, fetal kidney, and fetal heart.

**Table 1: Phase 1.5.1 Expression of 15603 w/ beta 2**

| **Tissue Type** | **Expression** |
|---|---|
| Artery normal | 0.6556 |
| Aorta diseased | 0.3871 |
| Vein normal | 0.1012 |
| Coronary SMC | 0 |
| HUVEC | 0 |
| Hemangioma | 28.1641 |
| Heart normal | 0.2302 |
| Heart CHF | 0.0759 |
| Kidney | 0.618 |
| Skeletal Muscle | 0.1668 |
| Adipose normal | 0.0932 |
| Pancreas | 0.172 |
| primary osteoblasts | 0.1037 |
| Osteoclasts (diff) | 0 |
| Skin normal | 0 |
| Spinal cord normal | 0 |
| Brain Cortex normal | 2.2203 |
| Brain-Hypothalamus normal | 0.4603 |
| Nerve | 0 |
| DRG (Dorsal Root Ganglion) | 0 |
| Breast normal | 0.3587 |
| Breast tumor | 0 |
| Ovary normal | 0.7609 |
| Ovary Tumor | 0 |
| Prostate Normal | 0.0355 |
| Prostate Tumor | 0.1041 |
| Salivary glands | 0.0261 |
| Colon normal | 0.0164 |
| Colon Tumor | 0.0566 |
| Lung normal | 0.6053 |
| Lung tumor | 0.9175 |
| Lung COPD | 0.6978 |
| Colon IBD | 0.006 |
| Liver normal | 0 |
| Liver fibrosis | 0 |
| Spleen normal | 0.0303 |
| Tonsil normal | 0.0084 |
| Lymph node normal | 0 |
| Small intestine normal | 0.0894 |
| Skin-Decubitus | 0.3123 |
| Synovium | 0.0708 |
| BM-MNC | 0 |
| Activated PBMC | 0 |
| Neutrophils | 0 |
| Megakaryocytes | 17.3972 |
| Erythroid | 0 |

**Table 2: 15603 Expression in Vessel Panel 2.1**

| **Tissue Type** | **Expression** |
|---|---|
| Coronary SMC | 0 |
| Huvec NS | 0 |
| Huvec Shear/static pooled | 0 |
| H/Adipose/PIT 695 | 0.1151 |
| H/Internal Mam Artery/Normal/AMC 263 | 0 |
| H/Internal Mam Artery/Normal/AMC 347 | 0.3221 |
| H/Internal Mam Artery/Normal/AMC350 | 0 |
| H/Internal Mam Artery/Normal/AMC 352 | 0.2863 |
| H/Artery/Normal/PIT 1180 | 0.0511 |
| H/Artery/normal/AMC 150 | 0 |
| H/Artery/normal/PIT 912 | 0.0765 |
| H/Artery/normal/NDR 352 | 0.045 |
| H/Aorta/Diseased/PIT 710 | 0 |
| H/Aorta/Diseased/PIT 712 | 0 |
| H/Aorta/Diseased/PIT 732 | 0.1909 |
| H/Artery/Diseased/iliac/NDR 753 | 0 |
| H/Artery/Diseased/Tibial/PIT 679 | 0.1238 |
| H/Vein/Normal/PIT 1010 | 0.3255 |
| H/Vein/Normal/NDR 239 | 0.0477 |
| H/Vein/Normal/AMC 130 | 0.2581 |
| H/Vein/Normal/AMC 131 | 0 |
| H/Vein/Normal/AMC 137 | 0 |
| H/Vein/Normal/AMC 153 | 0.0661 |
| H/Vein/Normal/AMC 176 | 0.0345 |
| H/Vein/Normal/AMC 177 | 0 |
| H/Vein/Normal/AMC 178 | 0.1002 |
| H/Vein/Normal/AMC 182 | 0.0323 |
| H/Vein/Normal/AMC 190 | 0 |
| H/Vein/Normal/AMC 192 | 0 |
| H/Vein/Normal/AMC 195 | 0 |
| H/Vein/Normal/AMC 211 | 0 |
| | |
| M/Aorta/Normal/PRI 286 | 0 |
| M/Vein/Normal/PRI 328 | 0 |
| M/Vein/Normal/PRI 230 | 0 |
| M/Aorta/Diseased/CAR 1216 | 0 |
| M/Aorta/Diseased/CAR 1237 | 0 |
| M/Aorta/Diseased/CAR 1192 | 0 |
| M/Aorta/Diseased/CAR 1196 | 0 |
| M/Artery/Diseased/CAR 1174 | 6.3899 |
| M/Artery/Diseased/CAR 1175 | 0 |

**Table 3: Expression of 1506**

| | **Value** |
|---|---|
| HUVECs Stat | 0. |
| HUVECs LSS | 0. |
| | |
| HUVECs Stat | 0. |
| LSS | 0. |
| | |
| HUVECs Prolif | 0. |
| HUVECs Conf | 0. |
| HUVECs -GF | 0. |
| HUVECs IL-1 | 0. |
| | |
| HMVEC-Cardiac Prolif | 0. |
| HMVEC-Cardiac Conf | 0. |
| | |
| HMVEC-Cardiac Prolif | 0. |
| HMVEC-Cardiac Conf | 0. |
| | |
| HMVEC-Lung Prolif | 0. |
| HMVEC-Lung Conf | 0. |
| HMVEC-Lung -GF | 0. |
| | |
| HMVEC-Lung Prolif | 1. |
| HMVEC-Lung Conf | 0. |
| | |
| Aortic C4h | 0. |
| Aortic TNF | 0. |
| Aortic C14 | 0. |
| Aortic TNF | 0. |
| | |
| 293 | 0. |
| | |
| HUVEC Stat | 0. |
| HUVEC LSS | 0. |
| HOVEC Stat | 0. |
| HUVEC LSS | 0. |
| | |
| HUVEC T6 | 0. |
| | |
| HUVEC RDC | 0. |
| HUVEC RDH | 0. |
| | |
| HUVEC HuC | 0. |
| HUVEC HuH | 0. |
| | |
| Hemangioma | 12 |
| Hemangioma | 7. |

**Table 4: Expression of 15603 with β2 in the Angiogenesis Panel**

| **Tissue Type** | **Expression** |
|---|---|
| ONC 101 Hemangioma | 14.9885 |
| ONC 102 Hemangioma | 4.6615 |
| ONC 103 Hemangioma | 5.1902 |
| CHT 1273 Glioblastoma | 0.1909 |
| CHT 216 Glioblastoma | 0.0318 |
| CHT 501 Glioblastoma | 0-1578 |
| NDR 203 Normal Kidney | 0.2082 |
| PIT 213 Renal Cell Carcinoma | 0 |
| CHT 732 Wilms Tumor | 1.4853 |
| CHT 765 Wilms Tumor | 4.4871 |
| NDR 295 Skin | 0 |
| CHT 1424 Uterine Adenocarcinoma | 1.0911 |
| CHT 1238 Neuroblastoma | 0 |
| BWH 78 Fetal Adrenal | 0.0346 |
| BWH 74 Fetal Kidney | 0.2635 |
| BWH 4 Fetal Heart | 0.1023 |
| MPI 849 Normal Heart | 0 |
| CLN 796 Spinal Cord | 0 |

## Claims

1. A method of identifying a compound capable of treating an angiogenic disorder comprising:
i) contacting a candidate compound with the nucleic acid molecule of SEQ ID NO:3 as shown in figure 1; and
ii) assaying the ability of the compound to inhibit the expression of the nucleic acid molecule,
thereby identifying a compound capable of treating an angiogenic disorder.

2. A method of identifying a compound capable of treating an angiogenic disorder comprising:
i) contacting a candidate compound with a polypeptide encoded by the nucleic acid molecule of SEQ ID NO:3 as shown in figure 1 or a cell expressing the polypeptide encoded by the nucleic acid molecule of SEQ ID NO:3 as shown in figure 1; and
ii) assaying the ability of the compound to inhibit the ion channel activity of the polypeptide,
thereby identifying a compound capable of treating an angiogenic disorder.

3. A method according to claim 2, wherein said assay is a cell based assay and said candidate compound is contacted with a cell expressing said polypeptide.

4. A method according to claim 2, wherein said assay is a cell free assay.

5. A method according to claim 4, wherein said polypeptide is provided as an ion channel in a plasma membrane.

6. A method according to claim 5, wherein said assaying the ability of the compound to inhibit the ion channel activity of the polypeptide comprises measuring the flow of ions across said ion channel.

7. A method according to claim 6, wherein the flow of ions across said ion channel is measured by patch clamp recording.

8. A method according to any one of claims 1 to 7, further comprising confirming the ability of a test compound to inhibit the expression of the nucleic acid molecule of SEQ ID NO:3 as shown in figure 1 or to inhibit the polypeptide encoded by the nucleic acid molecule of SEQ ID NO:3 as shown in figure 1 in an animal model.

## Patentansprüche

1. Verfahren zum Identifizieren einer Verbindung, die in der Lage ist, eine Angiogenese-Störung zu behandeln, umfassend die Schritte:
(i) Kontaktieren einer Kandidatenverbindung mit dem Nukleinsäuremolekül SEQ ID NO:3, wie in Figur 1 gezeigt; und
(ii) Testen der Fähigkeit der Verbindung die Expression des Nukleinsäuremoleküls zu hemmen mit einem Assay;
dadurch Identifizieren einer Verbindung, die in der Lage ist, eine Angiogenese-Störung zu behandeln.

2. Verfahren zum Identifizieren einer Verbindung, die in der Lage ist, eine Angiogenese-Störung zu behandeln, umfassend die Schritte:
(i) Kontaktieren einer Kandidatenverbindung mit einem Polypeptid, das durch das Nukleinsäuremolekül SEQ ID NO:3, wie in Figur 1 gezeigt, kodiert wird, oder mit einer Zelle, die das Polypeptid expremiert, das durch das Nukleinsäuremolekül SEQ ID NO:3, wie in Figur 1 gezeigt, kodiert wird; und
(ii) Testen der Fähigkeit der Verbindung die Ionenkanalaktivität des Polypeptids zu hemmen mit einem Assay;
dadurch Identifizieren einer Verbindung, die in der Lage ist, eine Angiogenese-Störung zu behandeln.

3. Verfahren gemäß Anspruch 2, wobei der Assay ein zellbasierter Assay ist und die Kandidatenverbindung mit einer Zelle kontaktiert wird, die das Polypeptid expremiert.

4. Verfahren gemäß Anspruch 2, wobei der Assay ein zellfreier Assay ist.

5. Verfahren gemäß Anspruch 4, wobei das Polypeptid als ein Ionenkanal in einer Plasmamembran bereitgestellt wird.

6. Verfahren gemäß Anspruch 5, wobei das Testen der Fähigkeit der Verbindung, die Expression des Nukleinsäuremoleküls zu hemmen, mit einem Assay umfasst das Messen des Ionenflusses durch den Ionenkanal.

7. Verfahren gemäß Anspruch 6, wobei der Ionenfluss durch den Ionenkanal durch Patchclamp-Aufzeichnen gemessen wird.

8. Verfahren gemäß einem jeden der Ansprüche 1 bis 7, ferner umfassend das Bestätigen der Fähigkeit der Verbindung die Expression des Nukleinsäuremoleküls SEQ ID NO:3, wie in Figur 1 gezeigt, zu hemmen oder das Polypeptid, das durch das Nukleinsäuremolekül SEQ ID NO:3, wie in Figur 1 gezeigt, kodiert wird, zu hemmen in einem Tiermodell.

## Revendications

1. Procédé d'identification d'un composé capable de traiter un trouble angiogénique, comprenant :
i) la mise en contact d'un composé candidat avec la molécule d'acide nucléique de SEQ ID NO:3 telle que représentée sur la figure 1 ; et
ii) la conduite d'un essai d'aptitude du composé à inhiber l'expression de la molécule d'acide nucléique,
permettant ainsi d'identifier un composé capable de traiter un trouble angiogénique.

2. Procédé d'identification d'un composé capable de traiter un trouble angiogénique, comprenant :
i) la mise en contact d'un composé candidat avec un polypeptide codé par la molécule d'acide nucléique de SEQ ID NO:3 telle que représentée sur la figure 1 ou une cellule exprimant le polypeptide codé par la molécule d'acide nucléique de SEQ ID NO:3 telle que représentée sur la figure 1 ; et
ii) la conduite d'un essai d'aptitude du composé à inhiber l'activité des canaux ioniques du polypeptide,
permettant ainsi d'identifier un composé capable de traiter un trouble angiogénique.

3. Procédé selon la revendication 2, dans lequel ledit essai est un essai à base de cellules et ledit composé candidat est mis en contact avec une cellule exprimant ledit polypeptide.

4. Procédé selon la revendication 2, dans lequel ledit essai est un essai exempt de cellules.

5. Procédé selon la revendication 4, dans lequel ledit polypeptide est fourni en tant que canal ionique dans une membrane plasmatique.

6. Procédé selon la revendication 5, dans lequel ledit essai de l'aptitude du composé à inhiber l'activité de canal ionique du polypeptide comprend la mesure du flux d'ions à travers ledit canal ionique.

7. Procédé selon la revendication 6, dans lequel le flux d'ions à travers ledit canal ionique est mesuré par un enregistrement patch clamp.

8. Procédé selon l'une quelconque des revendications 1 à 7, comprenant en outre la confirmation de l'aptitude d'un composé d'essai à inhiber l'expression de la molécule d'acide nucléique de SEQ ID NO:3 telle que représentée sur la figure 1 ou à inhiber le polypeptide codé par la molécule d'acide nucléique de SEQ ID NO:3 telle que représentée sur la figure 1 dans un modèle animal.
